# EUROPEAN PATENT APPLICATION

(11) **EP 2 255 797 A2**
(43) Date of publication of application: **01.12.2010**
(21) Application number: 09713514.9
(22) Date of filing: 21.02.2009
(51) Int. Cl.: A61K 9/52, A61K 9/00, A61K 9/20, A61K 9/22, A61K 47/00

(54) **COMPOSITE PREPARATION**

(30) Priority: 22.02.2008 KR 20080016115
(71) Applicant: HanAll Biopharma Co., Ltd., Daejeon 306-120 (KR)
(72) Inventor: KIM, Sung Wuk, Seongnam-si Gyeonggi-do 463-802 (KR); JUN, Sung Soo, Seongnam-si Gyeonggi-do 463-777 (KR); JO, Young Gwan, Daejeon 305-330 (KR); KOO, Ja Seong, Daejeon 305-756 (KR); SUN, Sang Ouk, Gwangju 501-200 (KR); BAE, Jung-won, Seoul 152-880 (KR)
(74) Representative: Pohlman, Sandra M.
(86) International application number: PCT/KR2009/000832
(87) International publication number: WO 2009/104932

(57) **Abstract**

The present invention provides a combination preparation which comprises: a prior-release section comprising aspirin or a pharmaceutically acceptable salt thereof as a pharmacologically active component; and a delayed-release section comprising clopidogrel, an isomer thereof or a pharmaceutically acceptable salt thereof as a pharmacologically active component. The combination preparation of the present invention exhibits a far better effect in preventing platelet aggregation than does simultaneous oral therapy or treatment with the respective single preparations, and not only can it improve the patient's drug-taking compliance by administration once a day but it can also reduce the adverse reactions which follow long-term administration of aspirin. The combination preparation of the present invention is also advantageous in that it exhibits an outstanding effect in inhibiting blood platelet aggregation despite a reduction in the amount of aspirin ingested, and in that it converts clopidogrel resistance into susceptibility and prevents serious adverse reactions caused by clopidogrel resistance and in that it can be stored over the longer term since it is stable under common storage conditions.

## Description

### TECHNICAL FIELD

The present invention relates to a combination preparation containing clopidogrel and aspirin and a method for producing the same.

### BACKGROUND ART

Intravascular thrombi or emboli formed by platelet aggregation lead to cardiovascular diseases. The term "cardiovascular disease (CVD)" refers to the group of diseases caused by dysfunctional conditions of the heart and blood vessels. As aging progresses, cardiac muscles weaken, and foreign materials such as cholesterol and calcareous matters are accumulated in coronary arteries to narrow arterial blood vessels, thus making smooth blood circulation difficult. The consequent diseases, including hyperlipidemia, cerebral stroke, myocardial infarction, arteriosclerosis, angina pectoris and the like, are referred to as cardiovascular diseases. In addition, there is the incidence of serious diseases as well as an important problem for re-surgery due to post-surgery formation of thrombi on stents in the rapidly increasing number of stent inserted surgery patients.

Clopidogrel, generally known as methyl (+)-(S)-α-(o-chlorophenyl)-6,7-dihydrothieno[3,2-c]pyridine-5(4H)-acetate, specifically inhibits platelet aggregation mediated by adenosine diphosphate (ADP) which is known to play an important role in thrombu

s formation. Clopidogrel is orally administrated and metabolized in the liver to become an active metabolite. The active metabolite prevents binding of ADP to an ADP receptor by selectively and irreversibly modifying the ADP receptor present in the platelets. Further, the active metabolite inhibits the binding between fibrinogen and GPIIb/IIIa complex mediated by ADP and properly controls the amplification of platelet aggregation which is induced by ADP. Consequently, through these mechanisms, clopidogrel provides antiplatelet and antithrombotic effects. Once clopidogrel modifies an ADP receptor irreversibly in the platelet, its inhibitory activity against platelet aggregation continues for about 7 days corresponding to the life span of platelets.

Such effects of clopidogrel are by the action of the active metabolite of clopidogrel. That is, an enzyme responsible for the metabolism of clopidogrel in the liver is an important factor for the efficacy of clopidogrel. Clopidogrel was initially expected to be metabolized only by cytochrome P450 1A, but it was found that clopidogrel is converted into an active metabolite by the action of cytochrome P450 3A4 (Atorvastatin reduces the ability of clopidogrel to inhibit platelet aggregation: a new drug-drug interaction. Circulation 2003; 107:32-37). With the most recent research, it was further revealed that a cytochrome P450 2C19 enzyme is involved in the conversion of clopidogrel into an active metabolite thereof. In particular, this study has a high reliability by demonstrating the influence of cytochrome P450 2C 19 on therapeutic effects of clopidogrel upon administration thereof alone, unlike other previous studies which focus on the measuring of the influence of clopidogrel on drugs which are metabolized by the cytochrome P450 enzyme (Cytochrome P450 2C19 loss-of-function polymorphism is a major determinant of clopidogrel responsiveness in healthy subjects. Jean-Sebastein et al., The American Society of Hematology, Blood, 1 October 2006. Vol 108, Number 7.).

Meanwhile, one of the most effective drugs for preventing thrombus formation is aspirin. Aspirin, generically referred to as acetylsalicylic acid, has been widely used as an antipyretic and analgesic agent, and has recently been used as a platelet aggregation inhibitor. When it is administered at a low dose, aspirin irreversibly acetylates cyclo-oxygenase (COX) of platelets to block the synthesis of thromboxane A2 (TXA-2) which is a platelet aggregation inducer, whereby the formation of thrombi is prevented by arresting the underlying scheme of platelet aggregation at the early stage thereof. Clopidogrel blocks the ADP binding stage where platelets aggregate with each other. Such antithrombotic effects of aspirin are exerted in a manner that when it is administered at a dose of 20 to 30 mg/day, aspirin saturates and irreversibly binds to thromboxane A2 synthetase COX-1 in whole platelets. Even though it exhibits antithrombotic effects, aspirin is poorly absorbed in the stomach and is therefore used at least at a dose of 75 to 100 mg/day. However, persons with weak stomach complain of side effects associated with stomach damage even at such an aspirin dose. Accordingly, there has been proposed for a long time that aspirin should be medicated at a minimum dose if possible [Lancet, III (1979) 1213, Prostaglandins and Medicine 4(1980) 439]. Particularly, also as shown in the most recent publications, a dose of aspirin ranging from 20 to 40 mg exhibited the most effective anti-platelet aggregation effects [Drug Insight: Aspirin Resistance - Fact or Fashion, Carlo Patrono et al., Nat Clin Pract Cardiovasc Med. 2007; 4(1):42-50][Low-Dose Aspirin in TIA and Thrombotic Stroke; Ask the Experts about Cardiovascular from Medscape Internal Medicine; Gerald W. Smetana, MD Division of General Medicine and Primary Care, Beth Israel Deaconess Medical Center, Harvard Medical School]. For the above-mentioned applications, an Aspirin Protect tablet (aspirin 100 mg, Bayer) in the form of an enteric tablet is available on the market.

In addition, antiplatelet effects of aspirin vary depending on the administration time, i.e. the expression time of medicinal efficacy in the body. The human body works according to a constant rhythm. In the daytime, the production of cyclo-oxygenase-2 (COX-2 enzyme) associated with inflammation is higher than cyclo-oxygenase-1 (COX-1 enzyme) which is mainly involved in the synthesis of thromboxane-A2. On the other hand, in the nighttime, thromboxane-A2 synthetase (COX-1 enzyme), which is constitutively present, predominantly works more than the COX-2 enzyme. Therefore, it is reasonable that aspirin is administered before bedtime. Then, aspirin shows the highest inhibitory activity on platelet aggregation by irreversible binding to the COX-1 enzyme, until platelets are killed or until fresh platelets are actively produced. That is, since platelet aggregation is active during sleeping at night, administration of aspirin immediately before bedtime can maximize anti-platelet aggregation effects [Administration Time-Dependent Effect of Aspirin on Blood Pressure in Untreated Hypertensive Patients, Hypertension 2003; 41; 1259-1267] [Aspirin at bedtime best time to cut blood pressure, May 15, 2002, The Annual Scientific Meeting of the American Society of Hypertension; Dr. Ramon D. Hermida of the University of Vigo, Spain] [Differing Administration time-dependent effects of low-dose aspirin on ambulatory blood pressure in dipper and non-dipper hypertensive patients, Ramon C Hermida et al., P-151.] (Ramon C Hermida et al., P-151].

In addition, platelet aggregation takes place in a manner that thromboxane-A2 is first produced, followed by ADP-mediated platelet aggregation. Accordingly, a reasonable medication scheme is in such a manner that aspirin, which inhibits the synthesis of thromboxane-A2, is allowed to work first, and then clopidogrel, which inhibits the action of ADP, is allowed to work.

According to the recent publications, about 50% of Asian races including Korean (about 30% in Western people) have a resistant gene against clopidogrel. Particularly, since most of patients with serious cardiac diseases, including patients with myocardial infarction or stent insertion, receive a combined therapy of aspirin and clopidogrel, there have been reported death cases of clopidogrel-resistant patients within one year due to aggravated formation of thrombi. This is because such patients are individuals who are deficient in cytochrome P450 2C19 which activates clopidogrel in the liver (Cytochrome P-450 polymorphisms and response to Clopidogrel, Jessica L. Mega, M.D., The New England Journal of Medicine, Jan 6, 2009). Naturally, aspirin is a substance which exerts a very useful specific action on clopidogrel-resistant individuals. Low-dose aspirin strongly activates a cytochrome P450 2C 19 enzyme, so when it is used in combination with clopidogrel which should be activated by cytochrome P450 2C19, it is reasonable to be medicated such that aspirin is released ahead of clopidogrel and enters first the liver [Isozyme-specific induction of low-dose aspirin on cytochrome P450 in healthy subjects. Clin Pharmacol Ther. 2003; 73(3):264-71]. However, combined therapies of aspirin and clopidogrel developed hitherto fail to make use of advantages of aspirin due to concomitant administration of these two drugs. Accordingly, it is reasonable that clopidogrel is administered 1 to 4 hours after the medication of aspirin.

As described above, clopidogrel specifically inhibits ADP-mediated platelet aggregation, and aspirin irreversibly acetylates cyclo-oxygenase (COX) to block the synthesis of thromboxane A2 (TXA-2) which is a platelet aggregation inducer, thus reducing platelets. Accordingly, aspirin prevents platelets in blood from adhering to each other, thus inhibiting platelet aggregation. There are many reports showing advantages of concurrent administration of clopidogrel and aspirin owing to such mutual complementary antiplatelet action thereof [Clopidogrel inhibits platelet aggregation in patients on aspirin with stable chronic angina pectoris. J. Cardiology.] [Combined therapy with clopidogrel and aspirin significantly increases the bleeding time through a synergistic antiplatelet action. J. Vascular surgery, 2002; 35: 1204-9][Addition of clopidogrel to aspirin in 45852 patients with acute myocardial infraction: randomized placebo-controlled trial. The Lancet. page 1607-1621]. Accordingly, there is released a therapeutic guideline that patients with acute coronary syndrome should receive combined administration of clopidogrel at a dose of 75 mg and aspirin at a dose of 75 to 325 mg, once a day [Physician desk Reference 57, "Plavix tablet"].

However, upon considering the action mechanisms of two drugs, it is difficult to anticipate a mutual complementary action of drugs simply by combined administration or combination preparation thereof.

Upon reviewing the action mechanisms of these drugs, it is most ideal that aspirin exerts first an anti-platelet aggregation action, and clopidogrel finishes the anti-platelet aggregation action which was not fulfilled by aspirin. In addition, aspirin exhibits the activation of cytochrome P450 2C 19 based on the theory of xenobiotics, so if clopidogrel is absorbed after aspirin is absorbed, the active metabolite of clopidogrel is further increased by the activated cytochrome P450 2C19 enzyme, thereby exhibiting superior effects. To this end, there is a need for the theory of chronotherapy whereby clopidogrel is absorbed after aspirin is released and absorbed.

However, under the current situation that low-dose aspirin is available only in the form of an enteric tablet due to the problem of gastrointestinal irritation of aspirin, it is apparent that, in order to achieve the above-mentioned advantages, administration of a clopidogrel preparation to a patient 4 hours (which corresponds to a period where aspirin reaches a peak blood level) after administration of an aspirin enteric preparation would lead to poor therapeutic effects due to lowering of the medication compliance resulting from an inconvenience to the patients.

Korean Patent No. 10-0295345 discloses a pharmaceutical composition having an anti-platelet aggregation activity, which contains clopidogrel and aspirin as active ingredients, but this patent focuses only on the synergism of drugs as a mixture of clopidogrel and aspirin.

US Patent No. 6015577 discloses a pharmaceutical composition for the prevention of clot formation and a process for preparing the same. This patent uses dipyridamole or mopidamol and aspirin as active ingredients of the pharmaceutical composition for the prevention of clot formation.

US Patent No. 5041430 discloses an oral anticoagulant/platelet inhibitor low dose formulation. This patent provides a composition containing warfarin as an anticoagulant and aspirin as a platelet inhibitor, and particularly states a production method for the prevention of instability between drugs.

Korean Patent Application Publication No. 2007-0021830 A1 discloses a process for preparing a sustained-release tablet containing cilostazol and aspirin.

Korean Patent Application Publication No. 1998-0001933 B1 and Korean Patent No. 10-0225017 disclose a method of producing buffered aspirin, for the inhibition of gastrointestinal hemorrhage which is a side effect of aspirin.

Conventionally, a combination preparation of clopidogrel and aspirin effective for the prevention and treatment of cardiovascular diseases has not been developed. To this end, there is a need for the development of such a combination preparation. In particular, it is necessary to solve the problems associated with gastrointestinal irritation side effects of aspirin and instability of the clopidogrel preparation, and there is a need for the development of a combination preparation excellent in therapeutic synergistic effects.

### DISCLOSURE OF THE INVENTION

### TECHNICAL PROBLEM

Therefore, the present invention is intended to provide a combination preparation of clopidogrel and aspirin, which is useful in the prevention and treatment of cardiovascular diseases.

Further, the present invention is intended to provide a method for the prevention and treatment of cardiovascular diseases.

### TECHNICAL SOLUTION

The present invention provides a combination preparation including a prior-release compartment containing aspirin or a pharmaceutically acceptable salt thereof as a pharmacologically active ingredient, and a delayed-release compartment containing clopidogrel, an isomer thereof or a pharmaceutically acceptable salt thereof as a pharmacologically active ingredient.

The prior-release compartment refers to a compartment whose active ingredient is released ahead of a delayed-release compartment in the combination preparation of the present invention, and the delayed-release compartment refers to a compartment whose active ingredient is released at a certain time interval after the release of the active ingredient of the prior-release compartment.

As used herein, the term "time interval" means that there is a difference in the time between aspirin and clopidogrel in order to achieve that they are respectively released and absorbed to thereby exert medicinal efficacy thereof.

The combination preparation of the present invention may be a combination preparation wherein aspirin is immediately released after oral administration of the preparation and is absorbed in the gastrointestine, and clopidogrel is released at a time-lag interval of about 5 to 120 minutes after oral administration of the preparation. The combination preparation is a preparation wherein clopidogrel is preferably released at a time-lag interval of about 15 to 90 minutes, and more preferably about 15 to 60 minutes.

As used herein, the term "immediate-release (or fast-disintegrating)" means that the dissolution of a drug ingredient is finished within about 15 minutes under the aqueous solution environment, and the term "time-lag interval" refers to the time taken to achieve the dissolution of 40% by weight of an active ingredient in the formulation under individual dissolution conditions, when a dissolution profile test is carried out *in vitro.*

When the combination preparation of the present invention is orally administered, aspirin is immediately released and absorbed in the gastrointestine.

As used herein, the term "release completion" or "dissolution completion" means that 85% or more of the active ingredient is released.

Specifically, the combination preparation of the present invention is configured such that aspirin is rapidly released and immediately absorbed in the gastrointestine after oral administration, thereby minimizing gastrointestinal irritation, and clopidogrel is delayed-released, whereby the release of clopidogrel is initiated after the release and absorption of aspirin are complete.

The combination preparation of the present invention may contain 0.01 to 20 parts by weight of aspirin relative to 1 part by weight of clopidogrel, and preferably 0.3 to 10 parts by weight of aspirin. If a content of aspirin is lower than 0.01 parts by weight, it may be difficult to achieve the anti-platelet aggregation action of aspirin. On the other hand, if a content of aspirin is higher than 20 parts by weight, therapeutic effects may be decreased due to the synthesis of prostaglandin being inhibited to thereby exhibit a platelet aggregation action.

The pharmaceutically acceptable salt of clopidogrel may be clopidogrel hydrogen sulphate or clopidogrel besylate.

In the combination preparation of the present invention, the aspirin-containing prior-release compartment may further contain an immediate-release material to increase a release rate of a drug, in addition to aspirin. The immediate-release material may be at least one selected from a disintegrant, a foaming agent and a buffer. The term "release" means that an active ingredient is disintegrated and dissolved. Solubility of aspirin in water or acid is not poor, so the disintegrated aspirin is rapidly dissolved. Therefore, immediate release of aspirin can be achieved by increasing a disintegration rate of a preparation using the disintegrant. Further, the disintegration rate of the preparation can be increased by using the foaming agent in combination with aspirin. The buffer, which is an alkaline material, is administered simultaneously with aspirin to thereby decrease the acidity of gastric contents. At the same time, the buffer reacts with aspirin to form a soluble salt, thereby increasing the solubility of aspirin. Therefore, immediate release of aspirin can be achieved by increasing the solubility of aspirin using the buffer. That is, the disintegrant and the foaming agent increase the disintegration rate of the preparation to thereby enhance the solubility of aspirin so that immediate release of aspirin becomes possible. The buffer is intended to increase the solubility of aspirin so that immediate release thereof is possible. In the present invention, the release rate of aspirin can be increased by using each of the disintegrant, the foaming agent, and the buffer, or a mixture of two or more thereof.

Examples of the disintegrant may include starches or modified starches such as sodium starch glycolate, corn starch, potato starch, and pregelatinized starch, clays such as bentonite, montmorillonite, and veegum, celluloses such as microcrystalline cellulose, hydroxypropylcellulose, and carboxymethylcellulose, algins such as sodium alginate, and alginic acid, crosslinked celluloses such as croscarmellose sodium, gums such as guar gum, and xanthan gum, and crosslinked polymers such as crosslinked polyvinylpyrrolidone (crospovidone). These materials may be used alone or in a combination of two or more thereof.

Examples of the foaming agent may include carbonate-containing inorganic materials and organic acids.

Examples of the carbonate-containing inorganic material may include sodium hydrogen carbonate, sodium carbonate, calcium carbonate, potassium carbonate, magnesium carbonate, calcium hydrogen carbonate and potassium hydrogen carbonate. These materials may be used alone or in a combination of two or more thereof.

Examples of the organic acid may include citric acid, hydrochloric acid, lactic acid, phosphoric acid, propionic acid, sulfuric acid, tartaric acid, fumaric acid, and malic acid. These materials may be used alone or in a combination of two or more thereof.

The buffer may be at least one selected from calcium carbonate, sodium dihydrogen phosphate, sodium monohydrogen phosphate, sodium glutamate, potassium citrate, sodium hydrogen carbonate, sodium citrate, sodium hydroxide, calcium phosphate, calcium hydrogen phosphate, and various salts thereof.

The aspirin-containing prior-release compartment of the present invention may further contain at least one additive selected from a diluent, a binder, a stabilizer and a film-coating agent.

Examples of the diluent may include calcium carbonate, calcium phosphate, cellulose, dextrin, dextrose, ethylcellulose, fructose, glyceryl palmitostearate, maltose, sucrose, starch, microcrystalline cellulose, lactose, glucose, mannitol, alginate, alkaline earth metal salts, clay, polyethylene glycol, dicalcium phosphate, and any combination thereof.

Examples of the binder may include alginic acid, carbomer, sodium carboxymethylcellulose, dextrin, ethylcellulose, hydroxyethylcellulose starch, hydroxyethylmethylcellulose, methylcellulose, polyethylene oxide, poloxamer, microcrystalline cellulose, mannitol, lactose, polyethylene glycol, polyvinylpyrrolidone, hydroxypropylmethylcellulose, hydroxypropylcellulose, natural gum, synthetic gum, copovidone, gelatin, and any combination thereof.

Examples of the glidant may include talc, stearic acid and salts thereof, sodium lauryl sulfate, hydrogenated vegetable oil, sodium benzoate, sodium stearyl fumarate, glyceryl monostearate, polyethylene glycol, and any combination thereof.

Examples of the stabilizer may include butylated hydroxytoluene (BHT), butylated hydroxyanisole (BHA), ascorbic acid, tocopherol, edetic acid (EDTA), and any combination thereof, which are conventionally used in the pharmaceutical industry.

Examples of the film-coating agent may include gelatin, methylcellulose, hydroxypropylcellulose, hydroxypropylmethylcellulose, polyethylene glycol, shellac, ethylcellulose, methylhydroxyethylcellulose, hydroxyethylcellulose, sodium carboxymethylcellulose, polyvinyl alcohol, polyvinylpyrrolidone, a vinylpyrrolidone/vinyl acetate polymer, an ethyl acrylate/methyl methacrylate/trimethyl ammonium chloride ethyl methacrylate copolymer (e.g., trade name: Eudragit RS or RL, Degussa, Germany), a methyl methacrylate/ethyl acrylate copolymer (e.g., trade name: Eudragit NE30D, Degussa, Germany), and polyvinylacetyl dimethylamino acetate. These materials may be used alone or in a combination of two or more thereof.

In the combination preparation of the present invention, the aspirin-containing prior-release compartment may be in the form of formulations such as a powder, a granule, a pellet, an uncoated tablet, a coated tablet, a fast-disintegrating tablet further containing a disintegrant in addition to an active ingredient, an effervescent tablet further containing a foaming agent in addition to an active ingredient, and/or a buffer tablet further containing a buffer in addition to an active ingredient. All of the formulations may be an immediate-release type.

As used herein, the term "fast-disintegrating" means that disintegration is finished within 15 minutes under the aqueous solution environment, and the term "fast-disintegrating tablet" refers to a tablet which is rapidly disintegrated. Solubility of aspirin in water or acid is not poor, so the dissolution of the drug is also achieved at the same rate as the disintegration rate. That is, at the time point where aspirin reaches the gastrointestine and is absorbed therein, aspirin is already present as an aqueous solution phase.

In the combination preparation of the present invention, the fast-disintegrating tablet or buffer tablet of aspirin can be prepared by lyophilization, spray drying, wet granulation, or direct compression, and the effervescent tablet can be prepared with the incorporation of a carbonate-containing inorganic material such as sodium hydrogen carbonate and an organic acid such as citric acid, in addition to the main ingredient.

The fast-disintegrating tablet may be a tablet which is prepared from the granules obtained by mixing aspirin and at least one selected from a binder, a disintegrant, and a glidant.

The binder, disintegrant, and glidant may be the same as defined above.

The effervescent tablet is a tablet which is prepared to have foamability by the incorporation of a foaming agent. The effervescent tablet generally has fast-disintegrating properties, and the fast disintegration thereof consequently leads to rapid dissolution of aspirin. The effervescent tablet may further contain at least one organic acid selected from citric acid, hydrochloric acid, lactic acid, phosphoric acid, propionic acid, sulfuric acid, tartaric acid, fumaric acid, and malic acid as a foaming agent, in addition to aspirin.

The buffer tablet refers to a tablet which is prepared to increase the solubility of a weak acidic drug aspirin by the incorporation of a buffer.

The foaming agent and buffer may be the same as defined above.

In the combination preparation of the present invention, the clopidogrel-containing delayed-release compartment can be obtained by coating an active ingredient with a hydrophilic polymer, an enteric polymer or a water-insoluble polymer, or by adding a controlled proportion of various pharmaceutically acceptable excipients to the active ingredient. The clopidogrel-containing delayed-release compartment may also be obtained by a production process such as high-pressure compression.

In the combination preparation of the present invention, the clopidogrel-containing delayed-release compartment may contain a release-controlling material as a pharmaceutically acceptable additive such that it is absorbed in the gastrointestine after a certain time-lag interval, in addition to clopidogrel. The release-controlling material may be at least one selected from a water-soluble polymer, a water-insoluble polymer, and an enteric polymer.

As used herein, the term "release-controlling material" refers to a material which controls the release timing and release volume of a pharmaceutical ingredient in order to enhance the medicinal efficacy.

The clopidogrel-containing delayed-release compartment may contain 0.01 to 10 parts by weight of the release-controlling material, based on 1 part by weight of clopidogrel. If a content of the release-controlling material is lower than 0.01 parts by weight, it may be difficult to control the release of the drug. On the other hand, if a content of the release-controlling material is higher than 10 parts by weight, effects of the drug may be decreased due to excessively delayed or extended release of the drug ingredient.

The term "water-soluble polymer" refers to a pharmaceutically acceptable water-soluble polymer which controls the release of a drug. Examples of the water-soluble polymer include a water-soluble cellulose ether selected from methylcellulose, hydroxypropylcellulose and hydroxypropylmethylcellulose, a water-soluble polyvinyl derivative selected from polyvinylpyrrolidone and polyvinyl alcohol, and an alkylene oxide polymer selected from polyethylene glycol and polypropylene glycol. These materials may be used alone or in a combination of two or more thereof.

Further, the term "water-soluble polymer" refers to a pharmaceutically acceptable water-insoluble polymer which controls the release of a drug. Examples of the water-insoluble polymer include a water-insoluble acrylate copolymer selected from polyvinyl acetate, a polymethacrylate copolymer, a poly(ethyl acrylate/methyl methacrylate) copolymer, an ethyl acrylate/methyl methacrylate/trimethylaminoethyl methacrylate copolymer (e.g., Eudragit RS 30D, Eudragit RL 30D, Evonik, Germany), ethylcellulose, cellulose ester, cellulose ether, cellulose acylate, cellulose diacylate, cellulose triacylate, cellulose acetate, cellulose diacetate, and cellulose triacetate, and a water-insoluble polyvinyl derivative such as polyvinyl acetate. These materials may be used alone or in a combination of two or more thereof.

The term "enteric polymer" refers to a polymer which is insoluble or stable under acidic conditions of less than pH 5, and is dissolved or degraded under specific pH conditions of pH 5 or higher. Examples of the enteric polymer may include an enteric cellulose derivative selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose and ethylhydroxyethylcellulose phthalate; an enteric acrylic acid copolymer selected from a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/ethyl methacrylate copolymer (e.g., trade name: Eudragit L 100 or Eudragit S, Degussa, Germany), a methacrylic acid/ethyl acrylate copolymer (e.g., trade name: Eudragit L 100-55, Degussa, Germany) and a methyl acrylate/methacrylic acid/octyl acrylate copolymer; an enteric maleic acid copolymer selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer and a butyl acrylate/styrene/maleic anhydride copolymer; and an enteric polyvinyl derivative selected from polyvinyl alcohol phthalate, polyvinylacetal phthalate, polyvinylbutyrate phthalate and polyvinylacetacetal phthalate. These materials may be used alone or in a combination of two or more thereof.

The clopidogrel-containing delayed-release compartment may further contain at least one additive selected from a diluent, a binder, a disintegrant, a glidant, a stabilizer, and a film-coating agent, as a pharmaceutically acceptable additive.

The diluent, binder, disintegrant, glidant, stabilizer, and film-coating agent may be the same as defined in the aspirin-containing prior-release compartment.

The combination preparation of the present invention may be prepared into a single formulation.

The combination preparation of the present invention can be prepared by simultaneously compressing clopidogrel together with coated particles or granules containing a release-controlling material selected from a water-soluble polymer, a water-insoluble polymer and an enteric polymer and obtained by a conventional coating method, and particles or granules of a granule composition containing immediate-release fast-disintegrating, effervescent or/and buffered aspirin, or by filling them in a capsule.

In addition, the combination preparation of the present invention may be any one of a pellet, an uncoated tablet, a coated tablet with a film-like coating layer, a multi-layered tablet, a press-coated tablet and a capsule.

The pellet may be made up of a clopidogrel coating layer formed of a delayed-release compartment on the sugar sphere surface, and an aspirin coating layer formed of a prior-release compartment enclosing the clopidogrel layer. A further coating layer may be formed between the clopidogrel coating layer and the aspirin coating layer of the pellet.

The multi-layered tablet may be in the form of a double-layered tablet including an aspirin layer formed of a prior-release compartment and a clopidogrel layer formed of a delayed-release compartment.

The multi-layered tablet may be in the form of a triple-layered tablet including an aspirin layer formed of a prior-release compartment, a clopidogrel layer formed of a delayed-release compartment, and a placebo layer which does not contain a pharmaceutical ingredient.

The combination preparation of the present invention may be formulated into a double-layered tablet or triple-layered tablet or more multi-layered tablet including a delayed-release granule layer containing clopidogrel and a prior-release granule layer containing aspirin.

The press-coated tablet may be made up of a clopidogrel inner core formed of a delayed-release compartment and an aspirin outer layer formed of a prior-release compartment. The clopidogrel inner core may further contain ethylcellulose as a release-controlling material, in addition to clopidogrel. Preferably, the clopidogrel inner core further contains hydroxypropylmethylcellulose as the release-controlling material, in addition to ethylcellulose.

The press-coated tablet may be in the form of a press-coated tablet including an inner core layer having a delayed-release granule layer containing clopidogrel and a prior-release outer layer containing aspirin.

The capsule may be in the form of a formulation containing a particle, granule, pellet, or tablet formed of a delayed-release compartment and a particle, granule, pellet, or tablet formed of a prior-release compartment. That is, the delayed-release compartment and the prior-release compartment may be respectively contained in the form of a particle, granule, pellet, or tablet in the same capsule.

A content of the coating layer in the coated tablet may be in the range of 0.5 to 20 % by weight, based on the total weight of the coated tablet. If a content of the coating layer is lower than 0.5% by weight, this may result in non-uniformity or variation of characteristics. On the other hand, if a content of the coating layer is higher than 20% by weight, this may result in simultaneous delayed-release of both ingredients from the tablet.

The combination preparation of the present invention can prevent the decomposition of drug ingredients due to interaction between the preparations by separately preparing the prior-release preparation of aspirin and the delayed-release preparation of clopidogrel. The delayed-release compartment of the present invention may be administered simultaneously with a commercially available fast-disintegrating aspirin preparation.

The preparation in accordance with the present invention, which is designed based on xenobiotic-chronotherapy, is not limited to the above-mentioned formulations. In the present invention, the range of additives such as the above-mentioned diluents is not limited to the use of the above-mentioned additives, and the additives may be used at a conventional dose which can be suitably selected by those skilled in the art.

In particular, aspirin and clopidogrel applied to the present invention are sensitive to water, so upon exposure to water, aspirin is hydrolyzed to become a salicylic acid, and clopidogrel is hydrolyzed to become an inactive carboxylic acid metabolite. As a consequence, no therapeutic effects are obtained upon administration thereof to the body. Therefore, production of pharmaceutical products and administration of additives should take into consideration water content and hygroscopicity.

Further, the present invention includes the formulation of a preparation by forming, if necessary, a film-like coating layer on the outer surface of the combination preparation. Here, the coating layer contains a film-forming agent, a film-forming aid or a mixture thereof.

The combination preparation of the present invention may be in the form of a kit including a delayed-release compartment and a prior-release compartment. Specifically, the kit includes a prior-release compartment; a delayed-release compartment; and a container for filling the prior-release compartment and the delayed-release compartment. The kit can be prepared in the form of a kit wherein a particle, granule, pellet, or tablet constituting the prior-release compartment is prepared, a granule, pellet, or tablet constituting the delayed-release compartment is additionally prepared, and the thus prepared two compartment materials are filled in a foil, blister, or bottle to prepare a dosage form for concurrent administration of different drugs.

In the present invention, a content of clopidogrel per unit preparation is in the range of 35.0 to 500.0 mg, and a content of aspirin is in the range of 10.0 to 1000.0 mg. More preferably, a content of clopidogrel per tablet is in the range of 75.0 to 300.0 mg, and a content of aspirin per tablet is in the range of 20 to 700 mg.

According to the combination preparation of the present invention, aspirin is first released and irreversibly acetylates cyclo-oxygenase of platelets to decrease the synthesis of thromboxane A2 which is a platelet aggregation inducer, thereby exhibiting a platelet aggregation inhibitory action, whereas clopidogrel prevents ADP from binding an ADP receptor by modifying the ADP receptor present in the platelets and clopidogrel also moderately controls the amplification of platelet aggregation which is induced by ADP.

The combination preparation of the present invention exhibits a complementary anti-platelet aggregation action by such a manner that aspirin is first released and absorbed and at a low dose, strongly activates a cytochrome P450 2C19 enzyme, and thereafter delayed-released clopidogrel can produce a clopidogrel active metabolite by the action of the activated cytochrome P450 2C19 enzyme.

The combination preparation of the present invention is preferably administered in the night in order to exhibit optimum anti-platelet aggregation effects, because platelet aggregation takes place actively in the nighttime, and cardiovascular diseases such as cerebral stroke and myocardial infarction also frequently take place during the night or dawn.

When it is formulated at a conventionally used dose, the combination preparation of the present invention exhibits superior anti-platelet aggregation effects to those exhibited by combined administration of conventional single preparations.

The combination preparation of the present invention contains aspirin as a fast-disintegrating preparation so that disintegration of aspirin after administration thereof is rapidly completed, and therefore aspirin is present in an aqueous solution state in the gastrointestine, whereby it is possible to maximize gastric absorption of the drug while minimizing the gastrointestinal disorders occurring due to contact of solid ingredients with stomach walls.

The combination preparation of the present invention exhibits superior effects through the application of the chronotherapeutic theory and xenobiotic theory to a formulation technique of a pharmaceutical preparation, as compared to individual or concurrent administration of conventional single preparations. In particular, even at a decreased dose of the active ingredient, the combination preparation of the present invention exhibits the anti-platelet aggregation effects equivalent to those of a conventional dose, while reducing side effects.

Further, through various formulations and production methods, the present invention can exhibit stable effects of drug ingredients even upon long-term storage of clopidogrel and aspirin which are physico-chemically labile.

The present invention is designed as a once-daily dosage form which can therefore improve the medication compliance of patients and reduce the production process time and costs of manufacturers as well as medication costs of patients.

Further, the present invention provides an anti-platelet aggregation method and/or a method for the prevention and treatment of cardiovascular diseases, by the administration of the combination preparation of the present invention.

The method for the prevention and treatment of cardiovascular diseases in accordance with the present invention includes administering to a subject the combination preparation of the present invention once a day between 5 p.m. and 11 p.m. The term "subject" refers to a mammal including a human, which includes a patient in need of administration of the combination preparation of the present invention. Therefore, the combination preparation of the present invention may be for evening administration (between 5 p.m. and 11 p.m.).

Further, the present invention provides a use of the combination preparation of the present invention for anti-platelet aggregation and for the treatment of cardiovascular diseases.

Further, the present invention provides an anti-platelet aggregation method or a method for the prevention and treatment of cardiovascular diseases, including administering aspirin to a subject, and then administering clopidogrel 5 to 120 minutes later.

Further, the present invention provides a method for preparing a chronotherapeutic combination preparation including a prior-release preparation containing aspirin and a delayed-release preparation containing clopidogrel.

Hereinafter, the method for preparing the combination preparation of the present invention will be specifically described according to individual steps.

Step 1 is a step of preparing a fast-disintegrating, effervescent, or/and buffered granule containing aspirin.

Step 2 is a step of obtaining a delayed-release granule or particle by subjecting clopidogrel together with a pharmaceutically acceptable conventional additive such as a water-soluble polymer, a water-insoluble polymer or an enteric polymer to conventional processes for producing oral solid preparations, for example, mixing, kneading, drying, and granulation.

Step 3 is a step of obtaining a preparation for oral administration by mixing the particles or granules prepared in each of Steps 1 and 2 with a pharmaceutically acceptable excipient and either simultaneously or sequentially compressing the mixture into a tablet or filling the mixture in a capsule, thereby obtaining a preparation for oral administration.

Step 1 may be carried out after Step 2, or Step 1 may be carried out simultaneously with Step 2.

The combination preparation of the present invention can be prepared according to the above procedure, and a formulation method will be described in more detail hereinafter.

### 1. Manufacturing of tablets

The particles or granules prepared in Step 1 are optionally coated with a polymer and then mixed with the granules prepared in Step 2, followed by compression into uniform weight, thereby preparing tablets. The resulting tablets may be film-coated for the purpose of improving the stability or shape, if necessary.

### 2. Manufacturing of multi-layered tablets

The granules prepared in Step 1 are optionally coated with a polymer, and dried. The dried granules are compressed with the granules prepared in Step 2 by using a multi-layered tablet press, thereby obtaining double-layered tablets. If necessary, triple or more multi-layered tablets may also be prepared by further adding placebo layer granules on the double-layered tablets. Coated multi-layered tablets may be prepared by coating the multi-layered tablets.

### 3. Manufacturing of press-coated tablets - I

The granules prepared in Step 2 are optionally coated with a polymer and dried, followed by compression into uniform weight. The resulting granules are used as an inner core optionally after performing further coating, and compressed with the granules prepared in Step 1 by using a core tablet press, thereby preparing press-coated tablets. Press-coated tablets with outer coating layer(hereinafter, refered to as coated press-coated tablet) may be prepared by coating the press-coated tablets.

### 4. Manufacturing of press-coated tablets - II

The granules prepared in Step 2 are optionally coated with a polymer and dried, followed by transfer to a hopper for inner core granules. The granules prepared in Step 1 were transferred to a hopper for outer layer granules, followed by compression into press-coated tablets by using a core tablet press. Coated press-coated tablets may be prepared by coating the press-coated tablets.

### 5. Manufacturing of capsules (granules)

The granules prepared in Step 1 are optionally coated with a release-controlling material, and dried. The dried granules together with the granules prepared in Step 2 may be placed in a capsule filling machine, and filled in capsules having a given size at an effective amount of each main ingredient, thereby preparing capsules.

### 6. Manufacturing of capsules (tablets)

The granules prepared in Step 1 are optionally coated with a release-controlling material, followed by compression into tablets. The granules prepared in Step 2 are optionally coated with a release-controlling material, followed by compression into tablets. The resulting tablets may be filled in capsules at a given amount taking into consideration effective amounts of active ingredients, thereby preparing capsules.

### 7. Manufacturing of pellet formulation

A clopidogrel ingredient may be coated on pellets of sugar particles or starch alone or pellets of a mixture thereof, followed by coating with an aspirin ingredient as an outer layer, thereby obtaining a pellet formulation. The pellets may be filled in a capsule or compressed into tablets, thereby securing a final formulation.

### 8. Manufacturing of kit

The preparation obtained in Step 1 and the preparation obtained in Step 2 may be filled in a foil, blister, or bottle to prepare a kit for concurrent administration of different drugs.

When the combination preparation of the present invention is orally administered once a day, particularly in the evening (before bedtime), synergistic effects due to chronotherapeutic administration can be obtained to allow for exertion of most excellent therapeutic or preventive effects on cardiovascular diseases. A dose of the combination preparation of the present invention to the human body is appropriately determined depending on absorptivity, inactivation rate and excretion rate of active ingredients in the body, age, sex and condition of patients, and the like. Preferably, a dose of the combination preparation is in the range of 75.0 to 300.0 mg of clopidogrel/day and 20.0 to 700 mg of aspirin/day.

### ADVANTAGEOUS EFFECTS

The combination preparation of clopidogrel and aspirin in accordance with the present invention may exhibit superior anti-platelet aggregation effects through the application of the chronotherapeutic theory and xenobiotic theory to a formulation technique of a pharmaceutical preparation, as compared to administration of individual ingredients as single drugs, or concurrent administration of single drugs of individual ingredients or administration of a simple combination preparation of individual ingredients, thereby exhibiting preventive and therapeutic effects against cardiovascular diseases.

Accordingly, the combination preparation of the present invention is excellent in clinical therapeutic effects and therefore can exhibit the same results even at a dose lower than a conventional normal dose. That is, a dose of main ingredients required for the prevention and treatment of cardiovascular diseases can be reduced and as a result, side effects are significantly reduced and production costs are curtailed.

The combination preparation of the present invention is also advantageous in that it exhibits outstanding anti-platelet aggregation effects despite a reduced dose of aspirin to be administered, in that it converts clopidogrel resistance into clopidogrel sensitivity and thereby prevents serious side effects due to clopidogrel resistance, and in that it can be stored over the longer term since it is stable under common storage conditions.

Finally, the combination preparation of the present invention can exhibit the above-mentioned superior effects by a once-daily administration through various formulations and can therefore improve the medication compliance of patients.

### DESCRIPTION OF THE DRAWINGS

FIG. 1 is a graph showing the dissolution profiles of aspirin in preparations of Comparative Example 1, and Preparation Examples 2, 3 and 5.
FIG. 2 is a graph showing the dissolution profiles of clopidogrel in preparations of Comparative Example 2, and Preparation Examples 8, 11 and 12.
FIG. 3 is a graph showing the dissolution profiles of aspirin in combination preparations of Examples 1, 6, 10 and 12.
FIG. 4 is a graph showing the dissolution profiles of clopidogrel in combination preparations of Examples 1, 6, 10 and 12.
FIG. 5 is a graph showing the dissolution profiles of clopidogrel in combination preparations of Examples 7, 8, 10 and 11.
FIG. 6 is a graph showing the dissolution profiles of clopidogrel and aspirin in combination preparations of Examples 15 and 18.
FIG. 7 is a graph showing the dissolution profiles of clopidogrel and aspirin in combination preparations of Examples 20 and 21.
FIG. 8 is a graph showing the dissolution profiles of clopidogrel and aspirin in combination preparations of Examples 22 and 23.
FIG. 9 is a graph showing the dissolution profiles of clopidogrel and aspirin in a combination preparation of Example 25.

### MODE FOR INVENTION

Advantages and features of the present invention and methods of achieving the same will become apparent from the detailed embodiments given below. This invention may, however, be embodied in different forms and should not be construed as being limited to the embodiments set forth herein. Rather, these embodiments are provided so that this disclosure will be thorough and complete, and will fully convey the scope of the invention to those skilled in the art. Therefore, the present invention should be defined by attached claims only.

### Preparation Example 1: Preparation of aspirin-containing prior-release compartment

According to the ingredients and contents shown in Table 1 below, aspirin, microcrystalline cellulose (Avicel PH101, FMC Biopolymer, USA), pregelatinized starch (Starch 1500G, Colorcon, USA), and colloidal silicon dioxide (Aerosil 200, Degussa, Germany) were mixed in a double cone mixer for 20 minutes to prepare a mixture. Meanwhile, stearic acid (Whawon Pharm. Co. Ltd., South Korea) was sieved through a No. 35 sieve, and then mixed with the above mixture for 4 minutes to obtain aspirin-containing immediate-release granules. The granules were then compressed into tablets using a tablet press (MRC-30, Sejong Machinery Co., Ltd., South Korea). Meanwhile, hydroxypropylmethylcellulose (Shin-Etsu Chemical Co., Ltd., Japan), polyethylene glycol 400 (Duksan Pure Chemical Co., Ltd., South Korea), talc (Whawon Pharm. Co. Ltd., South Korea), and titanium oxide (Whawon Pharm. Co. Ltd., South Korea) were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

### Preparation Examples 2: Preparation of aspirin-containing prior-release compartment

According to the ingredients and contents shown in Table 1 below, aspirin, lactose (DMV, Germany) and povidone granules (trade name: Ludipress, BASF, Germany), sodium hydrogen carbonate (Duksan Pure Chemical Co., Ltd., South Korea), and citric acid (Duksan Pure Chemical Co., Ltd., South Korea) were mixed in a double cone mixer for 20 minutes. Meanwhile, stearic acid was sieved through a No. 35 sieve, and then mixed with the above mixture for 4 minutes to obtain aspirin-containing immediate-release granules. The granules were then compressed into tablets using a tablet press (MRC-30, Sejong Machinery Co., Ltd., South Korea). Meanwhile, hydroxypropylmethylcellulose (Shin-Etsu Chemical Co., Ltd., Japan), polyethylene glycol 400, talc, and titanium oxide were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

### Preparation Example 3: Preparation of aspirin-containing prior-release compartment

According to the ingredients and contents shown in Table 1 below, aspirin, magnesium oxide, magnesium carbonate, calcium carbonate, and pregelatinized starch were mixed in a double cone mixer for 20 minutes. Meanwhile, stearic acid was sieved through a No. 35 sieve, and then mixed with the above mixture for 4 minutes to obtain aspirin-containing immediate-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

### Preparation Example 4: Preparation of aspirin-containing prior-release compartment

According to the ingredients and contents shown in Table 1 below, the preparation was carried out in the same manner as in Preparation Example 3, except that sodium starch glycolate (trade name: Primojel) was further added, in addition to aspirin, magnesium oxide, magnesium carbonate (Duksan Pure Chemical Co., Ltd., South Korea), calcium carbonate (Duksan Pure Chemical Co., Ltd., South Korea) and pregelatinized starch, followed by mixing in a double cone mixer for 20 minutes.

### Preparation Example 5: Preparation of aspirin-containing prior-release compartment

According to the ingredients and contents shown in Table 1 below, aspirin, mannitol (Pearlitol, Roquette, France), pregelatinized starch, and crospovidone (BASF, Germany) were mixed in a double cone mixer for 20 minutes. Meanwhile, stearic acid was sieved through a No. 35 sieve, and then mixed with the above mixture for 4 minutes to obtain aspirin-containing immediate-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

### Preparation Example 6: Preparation of aspirin-containing prior-release compartment

According to the ingredients and contents shown in Table 1 below, aspirin was coated with ethylcellulose (Ethocel, Colorcon, USA) dissolved in an ethanol/methylene chloride mixture (80 mg, 140 mg) using a fluidized bed coater (SFC-mini, Freund), followed by drying. The coated aspirin was mixed with mannitol, pregelatinized starch and crospovidone in a double cone mixer for 20 minutes. Meanwhile, stearic acid was sieved through a No. 35 sieve, and then mixed with the above mixture for 4 minutes to obtain aspirin-containing immediate-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture (ethanol 40 mg, methylene chloride 70 mg) to prepare a coating solution. The compressed tablets were placed in a coater (SFC-30, Sejong Machinery Co., Ltd., South Korea), and the coating solution was sprayed thereon to prepare film-coated tablets.

### Preparation Example 7: Preparation of clopidogrel-containing delayed-release compartment

According to the ingredients and contents shown in Table 1 below, clopidogrel hydrogen sulphate, low-hydrated microcrystalline cellulose (Vivapur 103, JRS), low-substituted hydroxypropylcellulose (LH-31, Shin-Etsu Chemical Co., Ltd.), and polyethylene glycol 6000 (PEG-6000, BASF) were sieved through a No. 20 sieve and then mixed in a double cone mixer for 20 minutes. The mixture was placed in a high-speed mixer (Speed mixer, Geumsung Chemical Machinery Co., Ltd., South Korea) and ethanol was added thereto, followed by kneading to prepare granules. The granules were dried in a hot-water dryer (Geumsung Chemical Machinery Co., Ltd., South Korea), followed by sieving. Stearic acid, which had been sieved through a No. 35 sieve, was added to the sieved material, followed by mixing for 4 minutes to obtain clopidogrel delayed-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

### Preparation Example 8: Preparation of clopidogrel-containing delayed-release compartment

According to the ingredients and contents shown in Table 1 below, clopidogrel hydrogen sulphate, low-hydrated microcrystalline cellulose, low-substituted hydroxypropylcellulose, and polyethylene glycol 6000 were sieved through a No. 20 sieve and then mixed in a fluidized bed granulator (SFC-mini, Freund, Japan) for 20 minutes. Meanwhile, hydroxypropylmethylcellulose was dissolved in 30 mg of ethanol to prepare a binding solution which was then kneaded with the above mixture in the fluidized bed granulator. When the drying process was complete after completion of the kneading process, ethylcellulose was dissolved in an ethanol/methylene chloride mixture (ethanol 80 mg, methylene chloride 140 mg), and coated on the dried material. The coated granules were mixed with pregelatinized starch and crospovidone for 10 minutes. Meanwhile, stearic acid was sieved through a No. 35 sieve, and then mixed with the above mixture for 4 minutes to obtain clopidogrel delayed-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture (ethanol 40 mg, methylene chloride 70 mg) to prepare a coating solution. The compressed tablets were placed in a coater (SFC-30, Sejong Machinery Co., Ltd., South Korea), and the coating solution was sprayed thereon to prepare film-coated tablets.

### Preparation Example 9: Preparation of clopidogrel-containing delayed-release compartment

According to the ingredients and contents shown in Table 1 below, the preparation was carried out in the same manner as in Preparation Example 8, except that hydroxypropylmethylcellulose was used instead of ethylcellulose.

### Preparation Example 10: Preparation of clopidogrel-containing delayed-release compartment

According to the ingredients and contents shown in Table 1 below, the preparation was carried out in the same manner as in Preparation Example 8, except that a mixture of hydroxypropylmethylcellulose and hydroxypropylmethylcellulose phthalate was used instead of ethylcellulose.

### Preparation Example 11: Preparation of clopidogrel-containing delayed-release compartment

According to the ingredients and contents shown in Table 1 below, clopidogrel hydrogen sulphate was placed in a fluidized bed coater, and a solution of ethylcellulose in an ethanol-methylene chloride mixture (ethanol 80 mg, methylene chloride 140 mg) was coated thereon. The coated granules were mixed with low-hydrated microcrystalline cellulose, low-substituted hydroxypropylcellulose, polyethylene glycol 6000 and colloidal silicon dioxide, which had been sieved through a No. 20 sieve, in a double cone mixer for 20 minutes. Meanwhile, stearic acid was sieved through a No. 35 sieve, and then mixed with the above mixture for 4 minutes to obtain clopidogrel delayed-release granules. The granules were then compressed into tablets using a tablet press (MRC-30, Sejong Machinery Co., Ltd., South Korea). Meanwhile, hydroxypropylmethylcellulose, polyethylene glycol 400, talc, and titanium oxide were dissolved in an ethanol/methylene chloride mixture (ethanol 40 mg, methylene chloride 70 mg) to prepare a coating solution. The compressed tablets were placed in a coater (SFC-30, Sejong Machinery Co., Ltd., South Korea), and the coating solution was sprayed thereon to prepare film-coated tablets.

### Preparation Example 12: Preparation of clopidogrel-containing delayed-release compartment

According to the ingredients and contents shown in Table 1 below, clopidogrel hydrogen sulphate, low-hydrated microcrystalline cellulose, low-substituted hydroxypropylcellulose, and polyethylene glycol 6000 were sieved through a No. 20 sieve and then mixed in a double cone mixer for 20 minutes. Stearic acid was sieved through a No. 35 sieve and then mixed with the above mixture for 4 minutes to obtain clopidogrel delayed-release granules. The granules were then compressed into tablets using a tablet press (MRC-30, Sejong Machinery Co., Ltd., South Korea). Meanwhile, polyvinyl alcohol, talc, titanium oxide, polyethylene glycol 400, and lecithin were dissolved in an ethanol/methylene chloride mixture to prepare a coating solution. The compressed tablets were placed in a coater (SFC-30, Sejong Machinery Co., Ltd., South Korea), and the coating solution was sprayed thereon to prepare film-coated tablets.

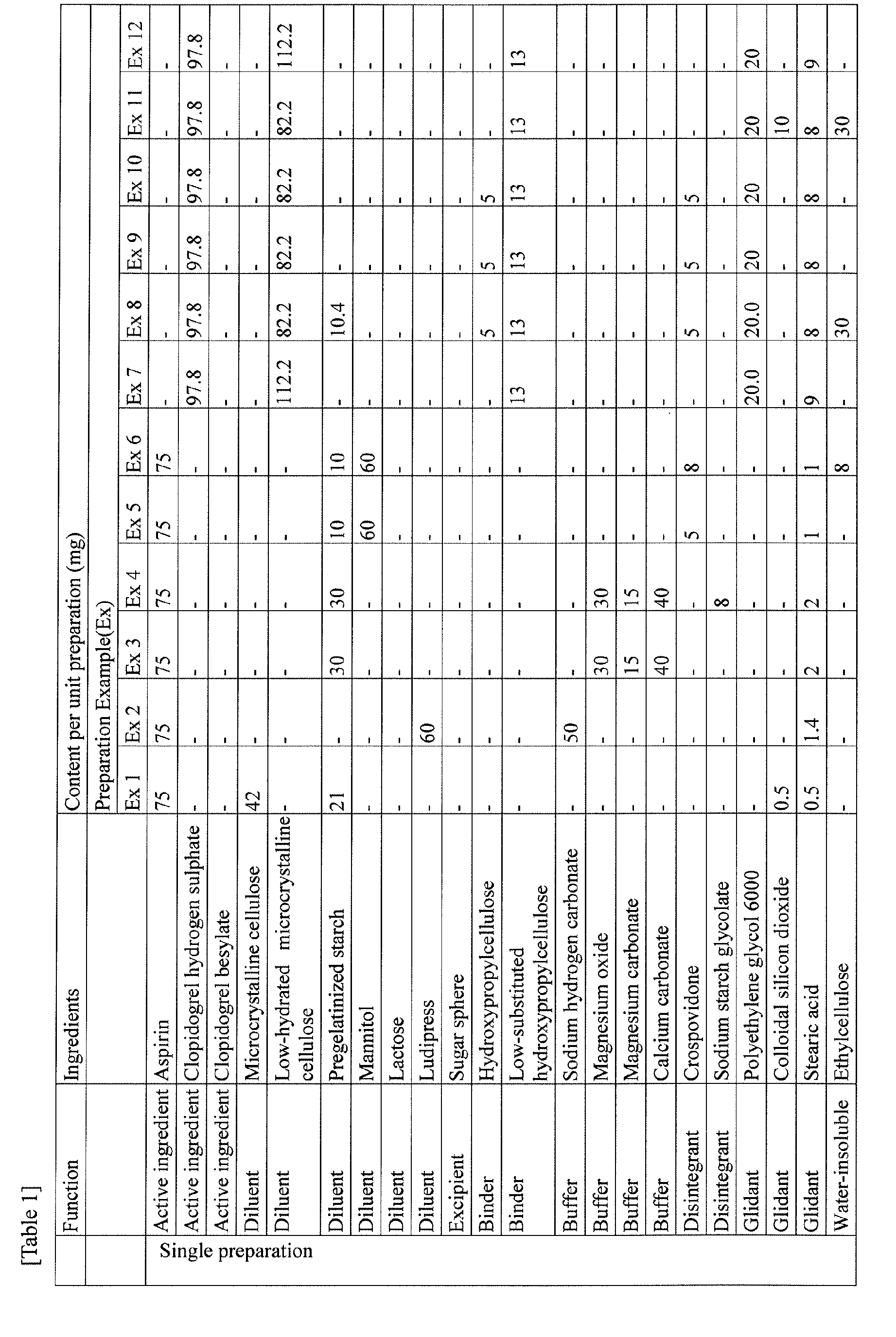

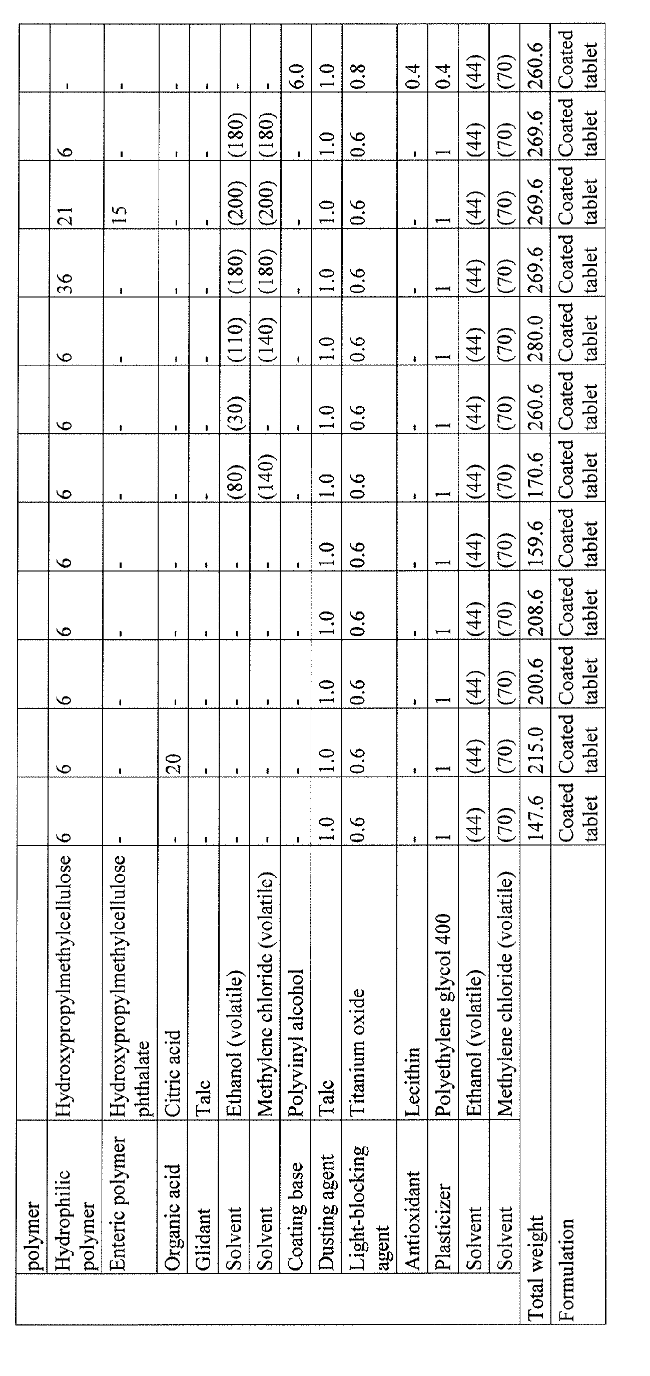

Hereinafter, embodiments of the combination preparation of the present invention will be provided.

### Example 1: Preparation of aspirin/clopidogrel-containing single tablets

### (1) Preparation of aspirin-containing granules

According to the ingredients and contents shown in Table 2 below, aspirin, magnesium oxide, magnesium carbonate, calcium carbonate, and pregelatinized starch were mixed in a double cone mixer for 20 minutes to obtain aspirin-containing prior-release granules.

### (2) Preparation of clopidogrel-containing granules

According to the ingredient composition shown in Table 2 below, clopidogrel hydrogen sulphate was placed in a fluidized bed coater, and a solution of ethylcellulose in an ethanol-methylene chloride mixture (50:50) was coated thereon. The coated granules were used as clopidogrel delayed-release granules.

### (3) Compression

The granules prepared in Processes (1) and (2) were mixed in a double cone mixer for 10 minutes. Meanwhile, stearic acid was sieved through a No. 35 sieve, and then mixed with the above mixture for 4 minutes. The resulting granules were then compressed into tablets using a tablet press (MRC-30, Sejong Machinery Co., Ltd., South Korea).

### (4) Coatings

Hydroxypropylmethylcellulose, polyethylene glycol 400, talc, and titanium oxide were dissolved in an ethanol/methylene chloride mixture to prepare a coating solution. The tablets of Process (3) were placed in a coater (SFC-30, Sejong Machinery Co., Ltd., South Korea), and the coating solution was sprayed thereon to prepare film-coated tablets.

### Example 2: Preparation of aspirin/clopidogrel-containing single tablets

According to the ingredients and contents shown in Table 2 below, the preparation was carried out in the same manner as in Example 1, except that clopidogrel besylate was used instead of clopidogrel hydrogen sulphate.

### Example 3: Preparation of aspirin/clopidogrel-containing single tablets

### (1) Preparation of aspirin-containing granules

According to the ingredients and contents shown in Table 2 below, aspirin, mannitol, pregelatinized starch, and crospovidone were mixed in a double cone mixer for 20 minutes to obtain aspirin-containing prior-release granules.

### (2) Preparation of clopidogrel-containing granules

According to the ingredient composition shown in Table 2 below, clopidogrel hydrogen sulphate was placed in a fluidized bed coater, and a solution of ethylcellulose in an ethanol-methylene chloride mixture (50:50) was coated thereon. The coated granules were used as clopidogrel delayed-release granules.

### (3) Compression

The granules prepared in Processes (1) and (2) were mixed in a double cone mixer for 10 minutes. Meanwhile, stearic acid was sieved through a No. 35 sieve, and then mixed with the above mixture for 4 minutes. The resulting granules were then compressed into tablets using a tablet press (MRC-30, Sejong Machinery Co., Ltd., South Korea).

### (4) Coatings

Hydroxypropylmethylcellulose, polyethylene glycol 400, talc, and titanium oxide were dissolved in an ethanol/methylene chloride mixture to prepare a coating solution. The tablets of Process (3) were placed in a coater (SFC-30, Sejong Machinery Co., Ltd., South Korea), and the coating solution was sprayed thereon to prepare film-coated tablets.

### Example 4: Preparation of aspirin/clopidogrel-containing single tablets

According to the ingredients and contents shown in Table 2 below, the preparation was carried out in the same manner as in Example 3, except that clopidogrel besylate was used instead of clopidogrel hydrogen sulphate.

### Example 5: Preparation of aspirin/clopidogrel-containing capsules

The final mixed granules prepared in Process (3) of Example 1 were filled in a No. 1 capsule to prepare a capsule formulation.

### Example 6: Preparation of aspirin/clopidogrel-containing double-layered tablets

### (1) Preparation of aspirin-containing granules

According to the ingredients and contents shown in Table 2 below, aspirin, magnesium oxide, magnesium carbonate, calcium carbonate, and pregelatinized starch were mixed in a double cone mixer for 20 minutes. After completion of the mixing process, stearic acid was sieved through a No. 35 sieve, and then mixed with the above mixture for 4 minutes to obtain aspirin-containing prior-release granules.

### (2) Preparation of clopidogrel-containing granules

According to the ingredients and contents shown in Table 2 below, clopidogrel hydrogen sulphate was placed in a fluidized bed coater, and a solution of ethylcellulose in an ethanol-methylene chloride mixture (50:50) was coated thereon. The coated granules were mixed with low-hydrated microcrystalline cellulose, low-substituted hydroxypropylcellulose, polyethylene glycol 6,000, and colloidal silicon dioxide (Aerosil 200 pharma, Deggusa), which had been sieved through a No. 20 sieve, in a double cone mixer for 20 minutes. Meanwhile, stearic acid was sieved through a No. 35 sieve, and then mixed with the above mixture for 4 minutes to obtain clopidogrel delayed-release granules.

### (3) Compression

The granules prepared in Processes (1) and (2) were respectively introduced into two inlets of a triple-layered tablet press (MRC-37, Sejong Machinery Co., Ltd., South Korea), followed by compression to obtain double-layered tablets.

### (4) Coating

Hydroxypropylmethylcellulose, polyethylene glycol 400, talc, and titanium oxide were dissolved in an ethanol/methylene chloride mixture to prepare a coating solution. The double-layered tablets of Process (3) were placed in a coater (SFC-30, Sejong Machinery Co., Ltd., South Korea), and the coating solution was sprayed thereon to obtain coated double-layered tablets.

### Example 7: Preparation of aspirin/clopidogrel-containing double-layered tablets

### (1) Preparation of aspirin-containing granules

According to the ingredients and contents shown in Table 2 below, aspirin, lactose/povidone granules (trade name: Ludipress), sodium hydrogen carbonate, and citric acid were mixed in a double cone mixer for 20 minutes. Meanwhile, stearic acid was sieved through a No. 35 sieve, and then mixed with the above mixture for 4 minutes to obtain aspirin-containing prior-release granules.

### (2) Preparation of clopidogrel-containing granules

According to the ingredients and contents shown in Table 2 below, clopidogrel hydrogen sulphate was placed in a fluidized bed coater, and a solution of ethylcellulose in an ethanol-methylene chloride mixture (50:50) was coated thereon. The coated granules were mixed with low-hydrated microcrystalline cellulose, low-substituted hydroxypropylcellulose, polyethylene glycol 6,000, and colloidal silicon dioxide, which had been sieved through a No. 20 sieve, in a double cone mixer for 20 minutes. Meanwhile, stearic acid was sieved through a No. 35 sieve, and then mixed with the above mixture for 4 minutes to obtain clopidogrel delayed-release granules.

### (3) Compression

The granules prepared in Processes (1) and (2) were respectively introduced into two inlets of a triple-layered tablet press (MRC-37, Sejong Machinery Co., Ltd., South Korea), followed by compression to obtain double-layered tablets.

### (4) Coating

Hydroxypropylmethylcellulose, polyethylene glycol 400, talc, and titanium oxide were dissolved in an ethanol/methylene chloride mixture to prepare a coating solution. The double-layered tablets of Process (3) were placed in a coater (SFC-30, Sejong Machinery Co., Ltd., South Korea), and the coating solution was sprayed thereon to obtain coated double-layered tablets.

### Example 8: Preparation of aspirin/clopidogrel-containing double-layered tablets

According to the ingredients and contents shown in Table 2 below, the preparation was carried out in the same manner as in Example 7, except that clopidogrel besylate was used instead of clopidogrel hydrogen sulphate.

### Example 9: Preparation of aspirin/clopidogrel-containing triple-layered tablets

According to the ingredient composition shown in Table 2 below, the preparation was carried out in the same manner as in Example 7, except that a placebo layer containing microcrystalline cellulose and stearic acid was introduced into an intermediate layer inlet of a triple-layered tablet press, followed by compression into triple-layered tablets.

### Example 10: Preparation of aspirin/clopidogrel-containing press-coated tablets

### (1) Preparation of aspirin-containing granules

According to the ingredients and contents shown in Table 2 below, aspirin, lactose/povidone granules (trade name: Ludipress), sodium hydrogen carbonate, and citric acid were mixed in a double cone mixer for 20 minutes. Meanwhile, stearic acid was sieved through a No. 35 sieve, and then mixed with the above mixture for 4 minutes to obtain aspirin-containing prior-release granules.

### (2) Preparation of clopidogrel-containing inner core tablets

According to the ingredients and contents shown in Table 2 below, clopidogrel hydrogen sulphate, low-hydrated microcrystalline cellulose, low-substituted hydroxypropylcellulose, and polyethylene glycol 6,000 were sieved through a No. 20 sieve and then mixed in a double cone mixer for 20 minutes. Stearic acid was sieved through a No. 35 sieve, and then mixed with the above mixture for 4 minutes to obtain clopidogrel delayed-release granules. The granules were then compressed into inner core tablets using a tablet press (MRC-30, Sejong Machinery Co., Ltd., South Korea). Meanwhile, ethylcellulose and hydroxypropylmethylcellulose were dispersed or dissolved in water to prepare a coating solution. The inner core tablets were placed in a coater (SFC-30, Sejong Machinery Co., Ltd., South Korea), and the coating solution was sprayed thereon to obtain coated inner core tablets.

### (3) Compression

The tablets of Process (2) as an inner core, and the granules of Process (1) as an outer layer were respectively introduced into inlets of a core tablet press (RUD-1, Kilian), followed by compression to obtain press-coated tablets.

### (4) Coating

Hydroxypropylmethylcellulose, polyethylene glycol 400, talc, and titanium oxide were dissolved in an ethanol/methylene chloride mixture to prepare a coating solution. The press-coated tablets of Process (3) were placed in a coater (SFC-30, Sejong Machinery Co., Ltd., South Korea), and the coating solution was sprayed thereon to obtain coated press-coated tablets.

### Example 11: Preparation of aspirin/clopidogrel-containing press-coated tablets

According to the ingredients and contents shown in Table 2 below, the preparation was carried out in the same manner as in Example 10, except that clopidogrel besylate was used instead of clopidogrel hydrogen sulphate.

### Example 12: Preparation of aspirin/clopidogrel-containing press-coated tablets

### (1) Preparation of aspirin-containing granules

According to the ingredients and contents shown in Table 2 below, aspirin, mannitol, pregelatinized starch, and crospovidone were mixed in a double cone mixer for 20 minutes. Meanwhile, stearic acid was sieved through a No. 35 sieve, and then mixed with the above mixture for 4 minutes to obtain aspirin-containing prior-release granules.

### (2) Preparation of clopidogrel-containing granules

According to the ingredients and contents shown in Table 2 below, clopidogrel hydrogen sulphate was placed in a fluidized bed coater, and a solution of ethylcellulose in an ethanol-methylene chloride mixture (50:50) was coated thereon. The coated granules were mixed with low-hydrated microcrystalline cellulose, low-substituted hydroxypropylcellulose, crospovidone, polyethylene glycol, and colloidal silicon dioxide, which had been sieved through a No. 20 sieve, in a double cone mixer for 20 minutes. Meanwhile, stearic acid was sieved through a No. 35 sieve, and then mixed with the above mixture for 4 minutes to obtain clopidogrel delayed-release granules.

### (3) Compression

Among the granules prepared in Processes (1) and (2), about 151.0 mg of the granules of Process (1) was set to be introduced into an outer layer inlet of a one-step core tablet press (OSDrC), and about 226.0 mg of the granules of Process (2) was set to be introduced into an inner core layer inlet, followed by compression to obtain press-coated tablets.

### (4) Coating

Hydroxypropylmethylcellulose, polyethylene glycol 400, talc, and titanium oxide were dissolved in an ethanol/methylene chloride mixture to prepare a coating solution. The press-coated tablets of Process (3) were placed in a coater (SFC-30, Sejong Machinery Co., Ltd., South Korea), and the coating solution was sprayed thereon to obtain coated press-coated tablets.

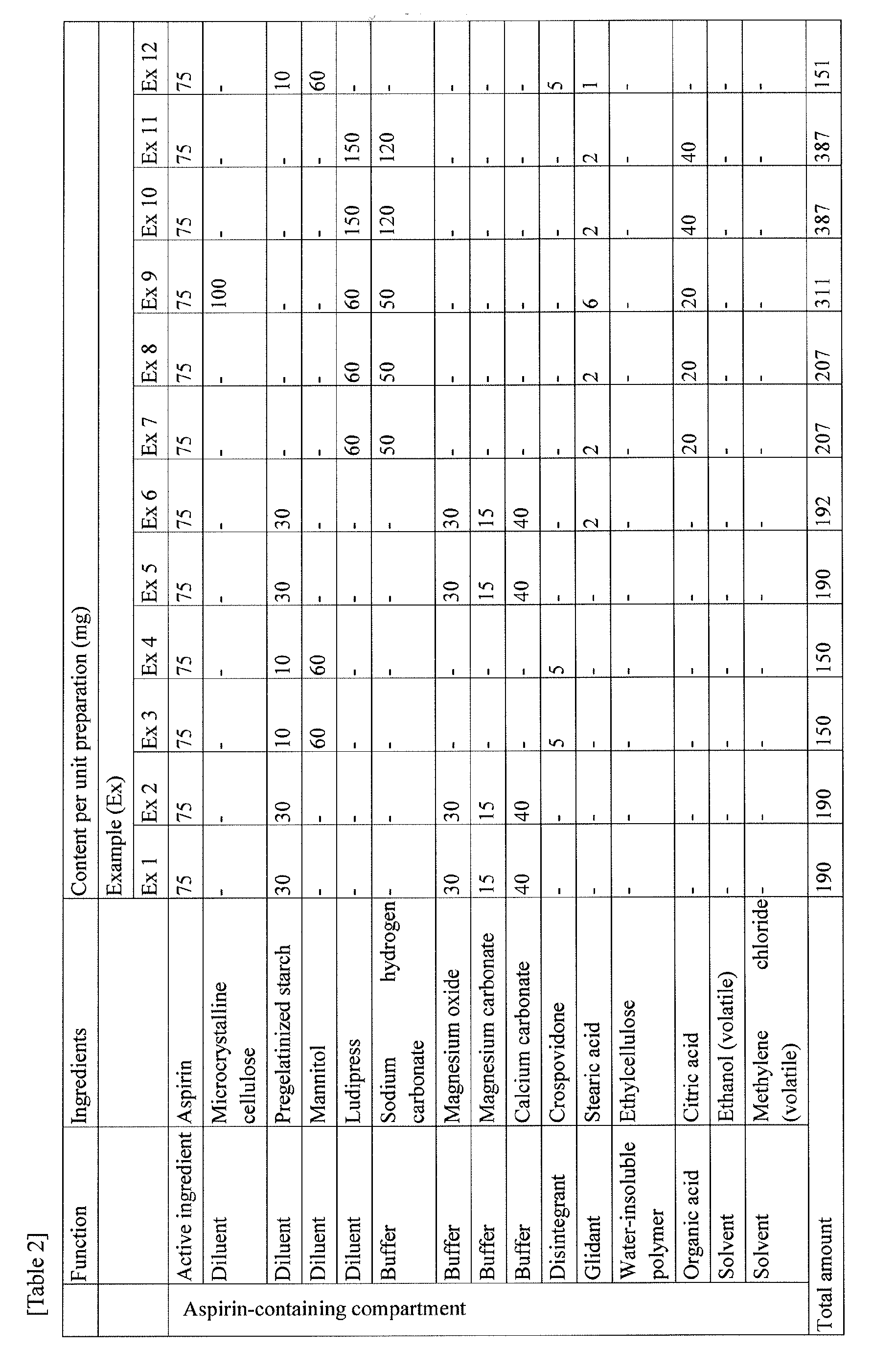

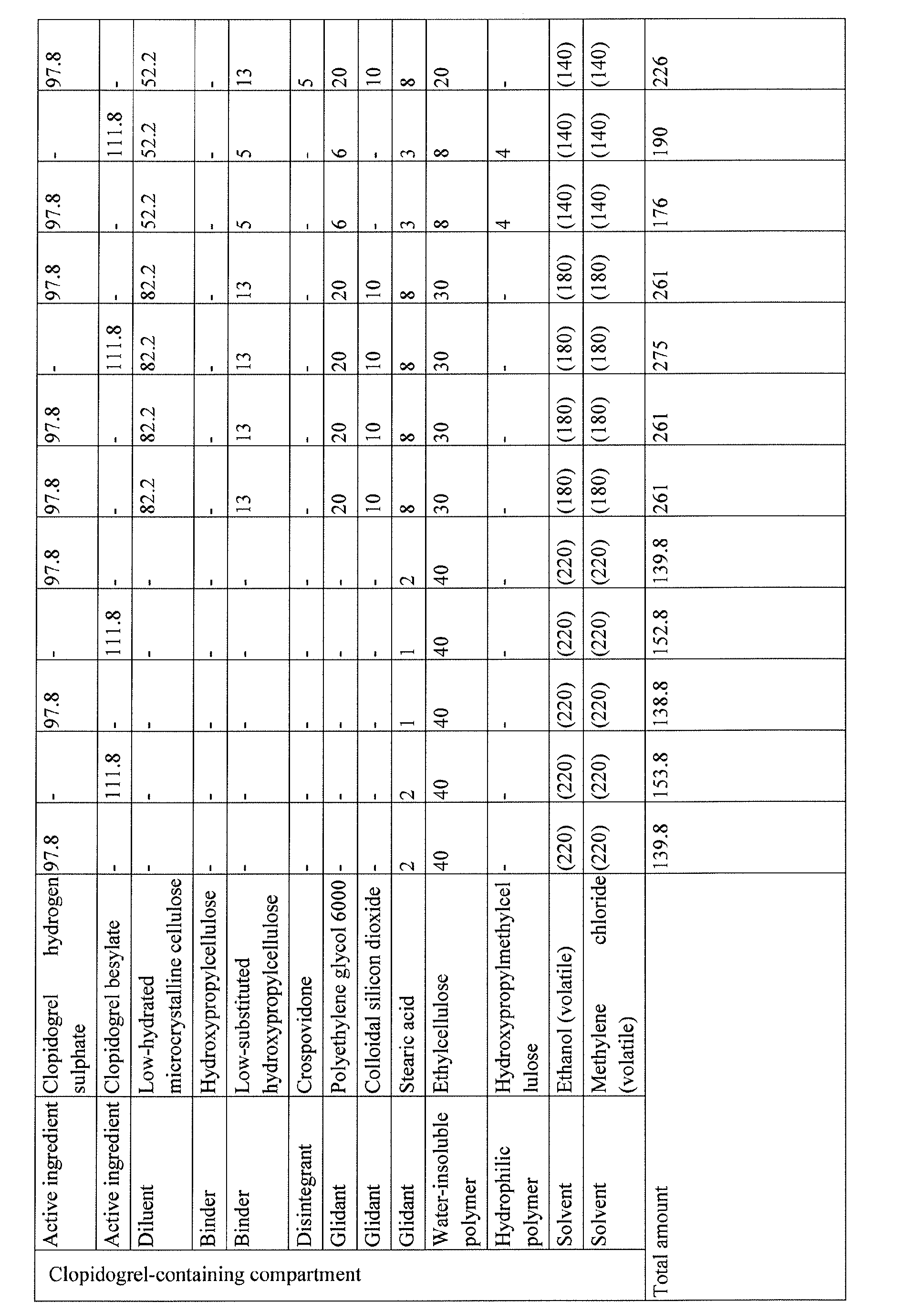

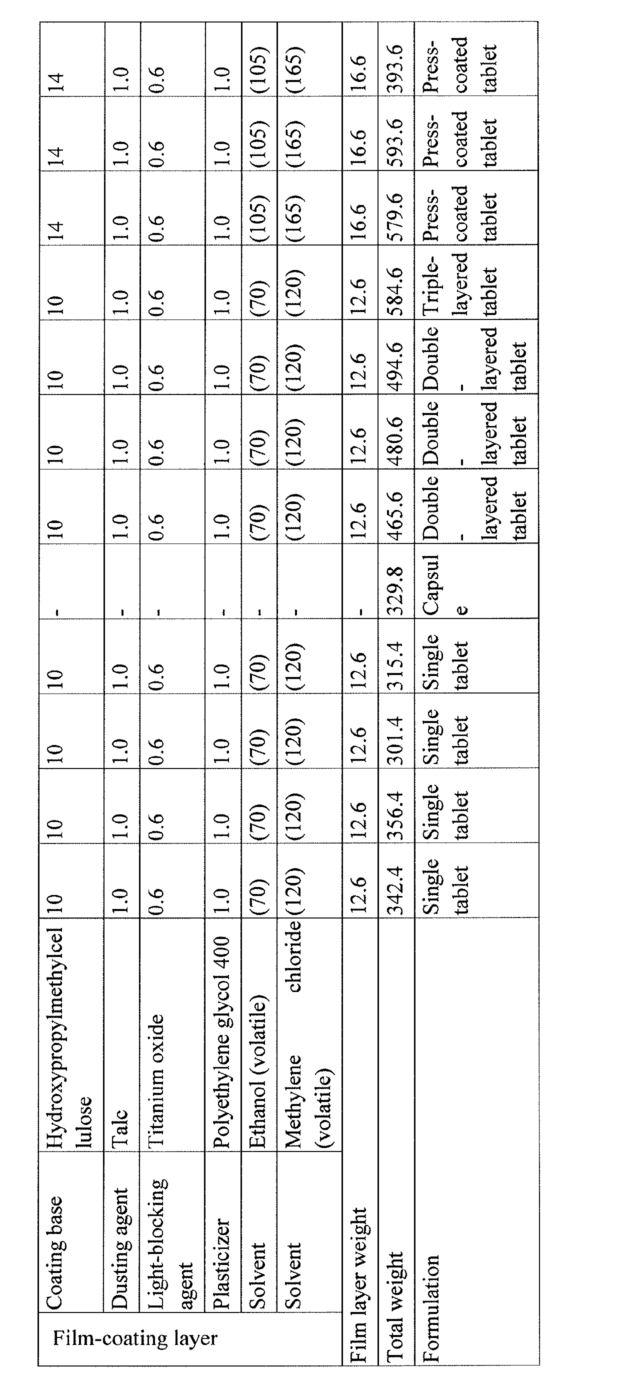

### Example 13: Preparation of clopidogrel/aspirin-containing pellets (capsules)

According to the ingredients and contents shown in Table 3 below, sugar spheres (400 µm to 600 µm) were placed in a fluidized bed coater. Meanwhile, clopidogrel hydrogen sulphate, hydroxypropylcellulose, and polyethylene glycol were dissolved in an ethanol-methylene chloride mixture, and the resulting solution was coated on the sugar spheres. For the purpose of layer division and dissolution rate control, hydroxypropylmethylcellulose was dissolved in 100 mg of ethanol, followed by further coating. Aspirin, talc, and hydroxypropylmethylcellulose were dissolved in an ethanol-methylene chloride mixture, and the resulting solution was coated on the clopidogrel-containing sugar spheres. Hydroxypropylmethylcellulose was dissolved in ethanol, followed by final coating. The thus prepared pellets were filled in a capsule to prepare a pellet preparation.

### Example 14: Preparation of clopidogrel/aspirin-containing capsules

### (1) Preparation of aspirin-containing tablets

According to the ingredients and contents shown in Table 3 below, aspirin, lactose/povidone granules (trade name: Ludipress), sodium hydrogen carbonate, and citric acid were mixed in a double cone mixer for 20 minutes. Meanwhile, stearic acid was sieved through a No. 35 sieve, and then mixed with the above mixture for 4 minutes to obtain aspirin-containing prior-release tablets.

### (2) Preparation of clopidogrel-containing tablets

According to the ingredients and contents shown in Table 3 below, clopidogrel hydrogen sulphate was placed in a fluidized bed coater, and a solution of ethylcellulose in an ethanol-methylene chloride mixture (50:50) was coated thereon. The coated granules were mixed with low-hydrated microcrystalline cellulose, low-substituted hydroxypropylcellulose, polyethylene glycol, and colloidal silicon dioxide, which had been sieved through a No. 20 sieve, in a double cone mixer for 20 minutes. Meanwhile, stearic acid was sieved through a No. 35 sieve, and then mixed with the above mixture for 4 minutes, followed by compression to obtain clopidogrel delayed-release tablets.

### 3 Filling

The tablets prepared in Processes (1) and (2) were introduced into an inlet of a capsule filling machine, and given amounts thereof were filled in blank capsules to prepare capsules.

### Examples 15 to 19: Preparation of combination preparations with changes in contents of clopidogrel and aspirin

According to the ingredients and contents shown in Table 3 below, the preparation was carried out in the same manner as in Example 6, except that the amount of aspirin to be used was changed.

### Examples 20 and 21: Preparation of combination preparations with changes in contents of aspirin and clopidogrel

According to the ingredients and contents shown in Table 3 below, the preparation was carried out in the same manner as in Example 7, except that amounts of main ingredients and additives of the clopidogrel hydrogen sulphate layer to be used were changed.

### Examples 22 to 24: Preparation of combination preparations with changes in contents of clopidogrel and aspirin

According to the ingredients and contents shown in Table 3 below, the preparation was carried out in the same manner as in Example 10, except that the amount of aspirin to be used was changed.

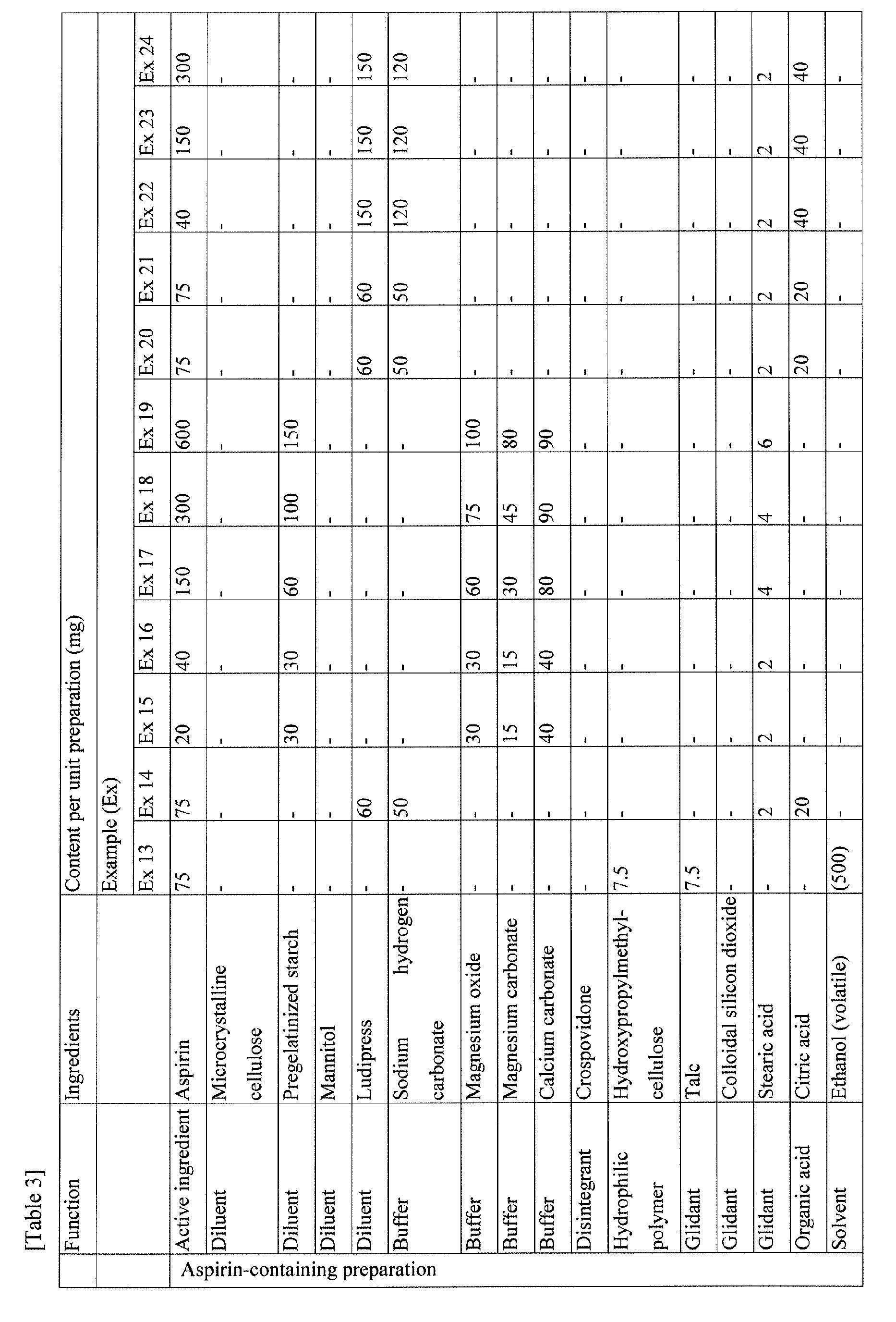

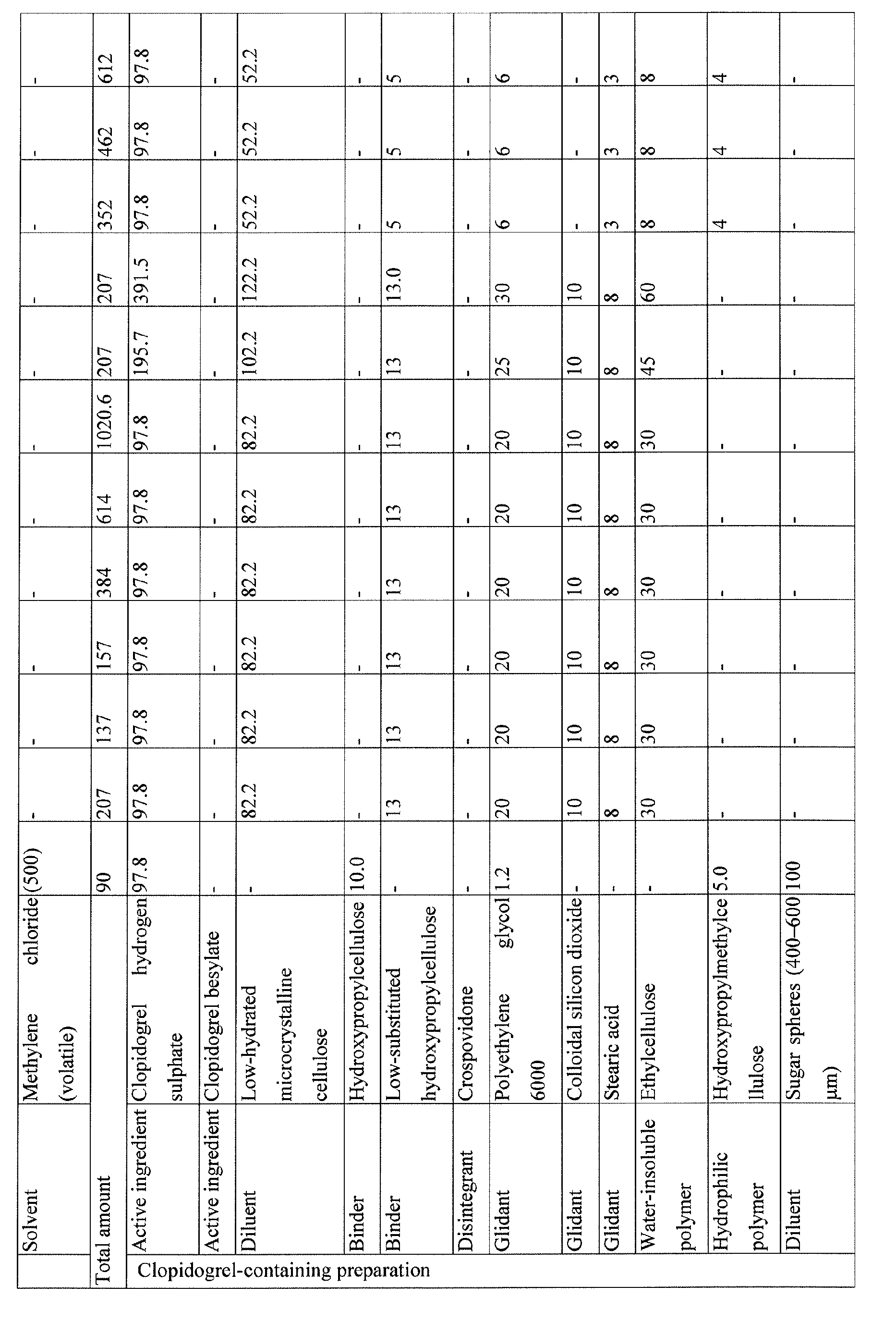

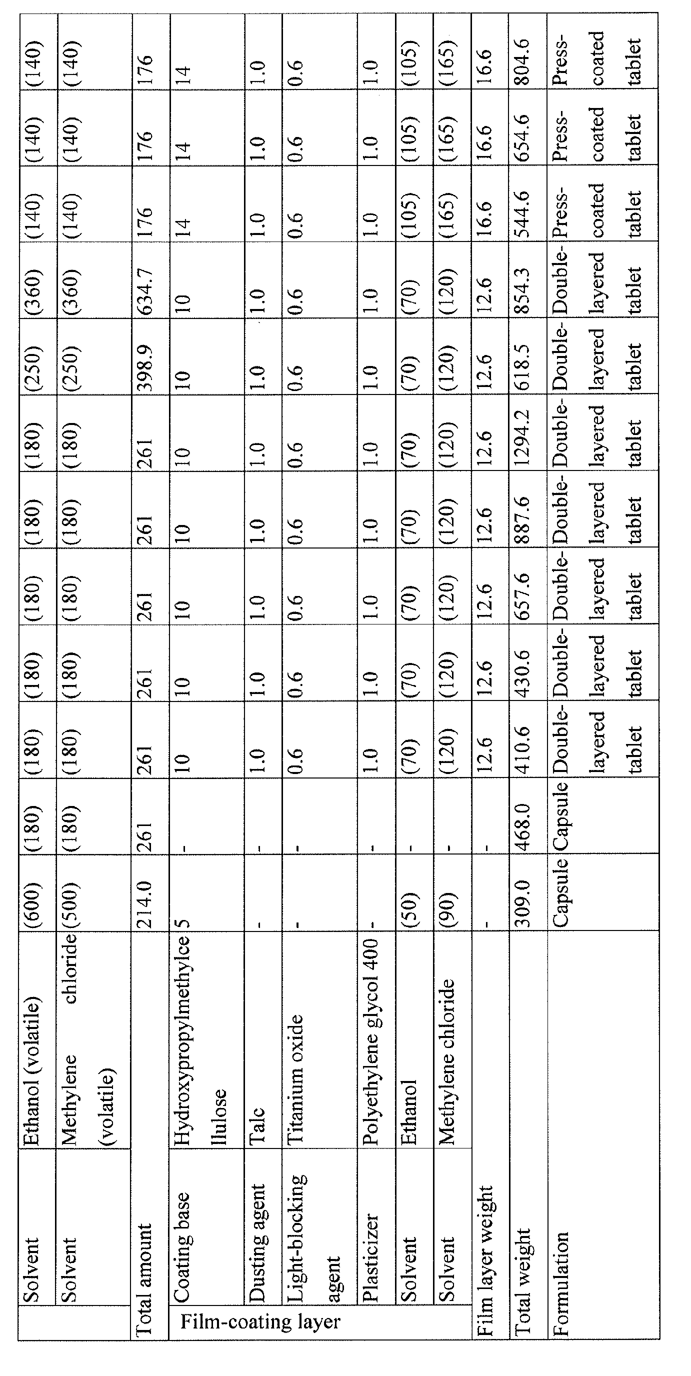

### Example 25: Preparation of aspirin/clopidogrel-containing triple-layered tablets

### (1) Preparation of aspirin-containing granules

122.5 g of aspirin, 192.5 g of microcrystalline cellulose, 62.5 g of pregelatinized starch, 30.0 g of alginic acid, 2.5 g of colloidal silicon dioxide, and 5.0 g of copovidone were respectively sieved through a No. 35 sieve and then mixed for 15 minutes. After completion of the mixing process, 2.5 g of stearic acid was sieved through a No. 35 sieve, and then added to the above mixture, followed by mixing for 3 minutes to prepare aspirin-containing granules.

### (2) Preparation of clopidogrel-containing granules

146.81 g of clopidogrel hydrogen sulphate, 142.69 g of microcrystalline cellulose, 22.5 g of polyethylene glycol, and 10.5 g of low-substituted hydroxypropylcellulose were sieved through a No. 25 sieve and then mixed for 15 minutes. Meanwhile, 4.5 g of hydroxypropylcellulose was dissolved in ethanol to prepare a binding solution which was then added to the above mixture, followed by kneading and drying. The dried granules were sieved through a No. 20 sieve. To the granules were added 15.0 g of copovidone and 34.5 g of sodium alginate which had been sieved through a No. 25 sieve, followed by mixing for 10 minutes. After completion of the mixing process, 13.5 g of stearic acid was sieved through a No. 35 sieve and then added to the above mixture, followed by mixing for 3 minutes to prepare clopidogrel-containing granules.

### (3) Preparation of placebo layer granules

105.0 g of ethylcellulose and 23.0 g of microcrystalline cellulose were sieved through a No. 25 sieve and then mixed for 10 minutes. After completion of the mixing process, 2.0 g of magnesium stearate was sieved through a No. 35 sieve and then added to the above mixture, followed by mixing for 3 minutes to prepare placebo layer granules.

### (4) Compression

The aspirin-containing granules of Process (1) were introduced into a first granule feeder of a triple-layered tablet press, the clopidogrel-containing granules of Process (2) were introduced into a second granule feeder of the triple-layered tablet press, and the placebo layer granules of Process (3) were introduced into a third granule feeder of the triple-layered tablet press. The triple-layered tablet press (MRC-37, Sejong Machinery Co., Ltd., South Korea) was set to ensure that 170.0 mg of aspirin granules, 260.0 mg of clopidogrel granules, and 130.0 mg of placebo layer granules are supplied to one tablet, followed by compression to obtain triple-layered tablets.

### (5) coating

5.0 mg of hydroxypropylcellulose, 15.0 mg of polyvinyl alcohol, 2.0 mg of polyethylene glycol, 2.0 mg of talc, and 0.5 mg of titanium oxide were dispersed or dissolved in purified water to prepare a coating solution. The compressed tablets of Process (4) were placed in a coater (SFC-30, Sejong Machinery Co., Ltd., South Korea), and the coating solution was sprayed and coated thereon to obtain coated triple-layered tablets.

### Example 26: Preparation of aspirin/clopidogrel-containing press-coated tablets

The clopidogrel-containing granules of Process (2) of Example 25 were compressed at a weight of 260.0 mg/tablet, which was then used as an inner core. The clopidogrel inner core tablets were introduced into an inner core feeder of a core tablet press (RUD-1, Kilian), and the aspirin-containing granules of Process (1) of Example 25 were introduced into an outer layer granule feeder at an amount of 340.0 mg/tablet, followed by compression into press-coated tablets. 5.0 mg of hydroxypropylcellulose, 15.0 mg of polyvinyl alcohol, 2.0 mg of polyethylene glycol, 2.0 mg of talc, and 0.5 mg of titanium oxide were dispersed or dissolved in purified water to prepare a coating solution. The press-coated tablets were placed in a coater (SFC-30, Sejong Machinery Co., Ltd., South Korea), and the coating solution was sprayed and coated thereon to obtain coated press-coated tablets.

### Example 27: Preparation of aspirin/clopidogrel-containing blister kits

The aspirin-containing granules of Process (1) of Example 7 and the clopidogrel-containing granules of Process (2) were respectively compressed into tablets using a rotary tablet press (MRC-30: Sejong), and the respective tablets were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Comparative Example 1: Clopidogrel single preparation

The currently commercially available preparation (PLAVIX, 75mg tablets, Sanofi) containing 97.875 mg of clopidogrel hydrogen sulphate (75 mg in terms of clopidogrel) was used as Comparative Example 1.

### Comparative Example 2: Aspirin single preparation

The currently commercially available enteric preparation (Aspirin Protect, Bayer Korea) containing 100 mg of aspirin was used as Comparative Example 2.

### Experimental Example 1: Disintegration test

The preparations obtained in Preparation Examples above were tested according to a disintegration method among general test methods described in the Korean Pharmacopoeia (8^{th} edition). The test conditions such as test fluids are as follows. In a specific test method, a disintegration tester was hung on a vertical axis member, placed in a beaker and gently moved upward and downward at 29 to 32 strokes per minute with amplitude of 53-57 mm. The movement of the tester was controlled such that, when the tester was moved down to the lowest position, the surface of a network disposed at the lower portion of the tester was 25 mm from the bottom of the beaker. Also, the amount of a test fluid in the beaker was adjusted such that, when the tester was moved down to the lowest position, the upper surface of the tester coincided with the surface of the fluid. The temperature of the fluid was maintained at 37 ± 2°C. As the test fluid, a simulated gastric fluid (pH: about 1.2) and a simulated intestinal fluid (pH: about 6.8) were used.

6 samples for each preparation example were taken, each sample was placed in the glass tube of the tester, and the tester was immersed in the test fluid in the beaker, the temperature and fluid volume of which had been controlled in advance. The tester was moved upward and downward for a given period of time and then the tester was carefully taken out of the test fluid. The state of the samples in the glass tube was observed. The results including disintegration time are given in Table 4.

**[Table 4]**

| Disintegration test results | | | | | |
|---|---|---|---|---|---|
| | Test group | Disintegration test | | | |
| | | Simulated gastric fluid (pH 1.2) | | Simulated intestinal fluid (pH 6.8) | |
| | | Disintegration finished | Disintegration time (mean, min) | Disintegration finished | Disintegration time (mean, min) |
| Control | Comparative Example 1 | X | No disintegration | O | 14.2 |
| | Comparative Example 2 | O | 18.4 | O | 17.2 |
| Aspirin- | Preparation | O | 4.3 | O | 4.2 |
| containing | Example 1 | | | | |
| preparation | Preparation Example 2 | O | 1.4 | O | 1.7 |
| | Preparation Example 3 | O | 2.2 | O | 2.9 |
| | Preparation Example 4 | O | 2.4 | O | 3.5 |
| | Preparation Example 5 | O | 0.9 | O | 1.3 |
| | Preparation Example 6 | O | 1.3 | O | 1.8 |
| Clopidogrel- | Preparation | O | 29.4 | O | 27.2 |
| containing | Example 7 | | | | |
| preparation | Preparation Example 8 | O | 41.5 | O | 41.8 |
| | Preparation Example 9 | O | 40.9 | O | 41.1 |
| | Preparation Example 10 | O | 51.9 | O | 43.7 |
| | Preparation Example 11 | O | 40.8 | O | 39.6 |
| | Preparation Example 12 | O | 63.1 | O | 61.5 |

As can be seen from the results of Table 4, the immediate-release aspirin tablets of Preparation Examples in accordance with the present invention exhibited rapid disintegration. In particular, Preparation Example 2 exhibited the occurrence of rapid disintegration in conjunction with profiles of effervescent disintegration. On the other hand, Aspirin Protect of Comparative Example 2 exhibited no disintegration for 2 hours in the first fluid of the disintegration test method, which is intended to be suitable for the enteric tablet standard. On the other hand, the disintegration test results showed that the overall disintegration time of the clopidogrel-containing preparations exhibited is slower than the aspirin-containing preparations. In particular, a slow disintegration time of Preparation Examples 10 and 12 in the disintegration test at a pH of 1.2 is believed to be due to the use of an enteric polymer and the presence of coating, respectively. From these experimental results, it was demonstrated that a prior-release compartment containing immediate-release aspirin and a delayed-release compartment containing clopidogrel can be prepared which are sought to be developed by the present invention.

### Experimental Example 2: Dissolution profile test

The preparations obtained in Preparation Examples and Examples above were subjected to a dissolution test under the conditions of simulated gastric fluid (pH 1.2) and purified water.

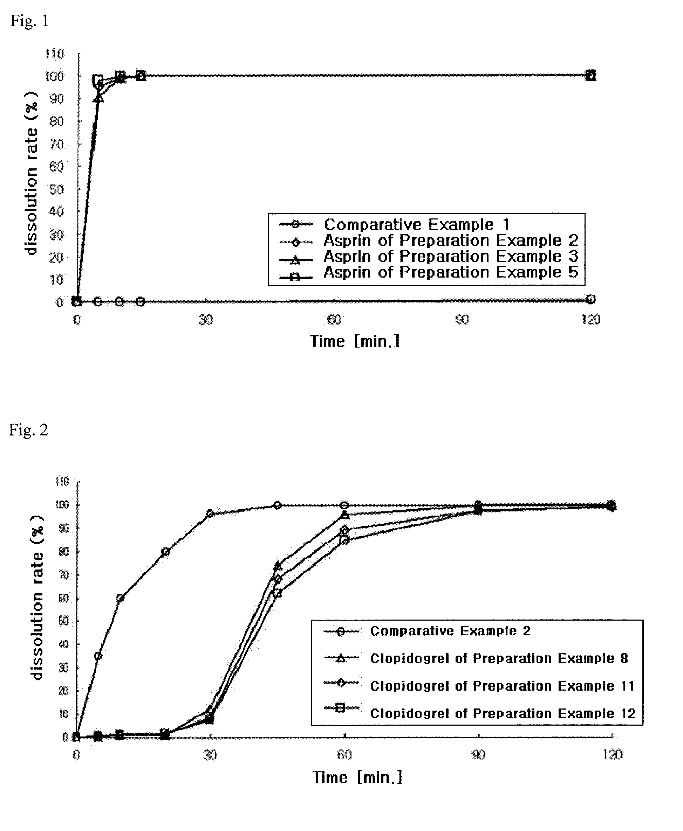

In a specific test method, 900 mL of purified water or simulated gastric fluid (a first fluid in a disintegration test method described in the Korean Pharmacopoeia), which was heated to 37±0.5°C, was placed in a basket and a dissolution test was performed at a paddle rotation speed of 50 rpm or 75 rpm. Six samples for each of Preparation Examples and Examples were taken, and one sample was placed in each basket. However, in the case of the capsule formulations, a sinker was used as an auxiliary device. After the start of dissolution, a given amount of the dissolution medium was taken at a given interval of time and analyzed to measure the dissolution rate. The measurement results are given in FIGS. 1 to 8.

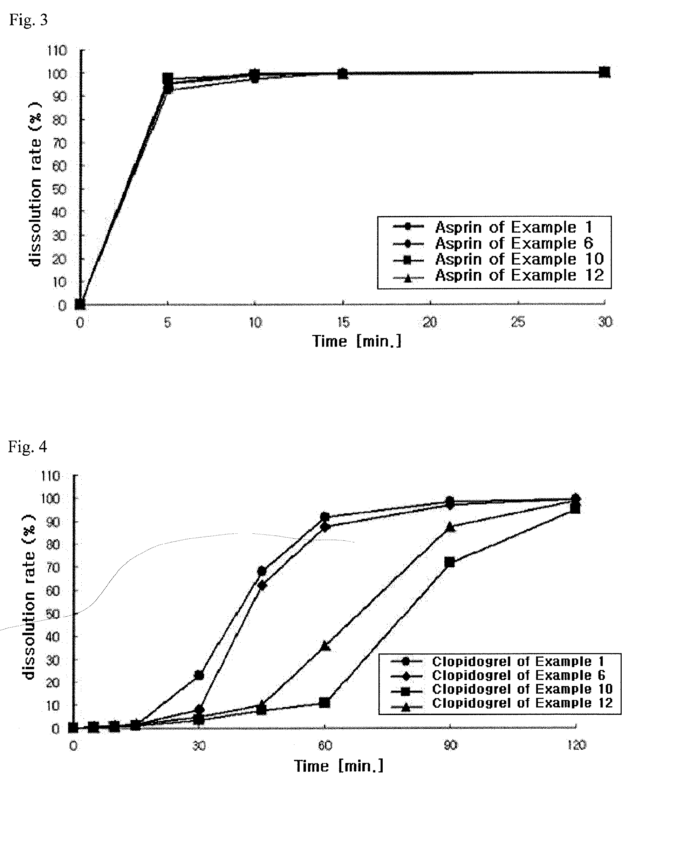

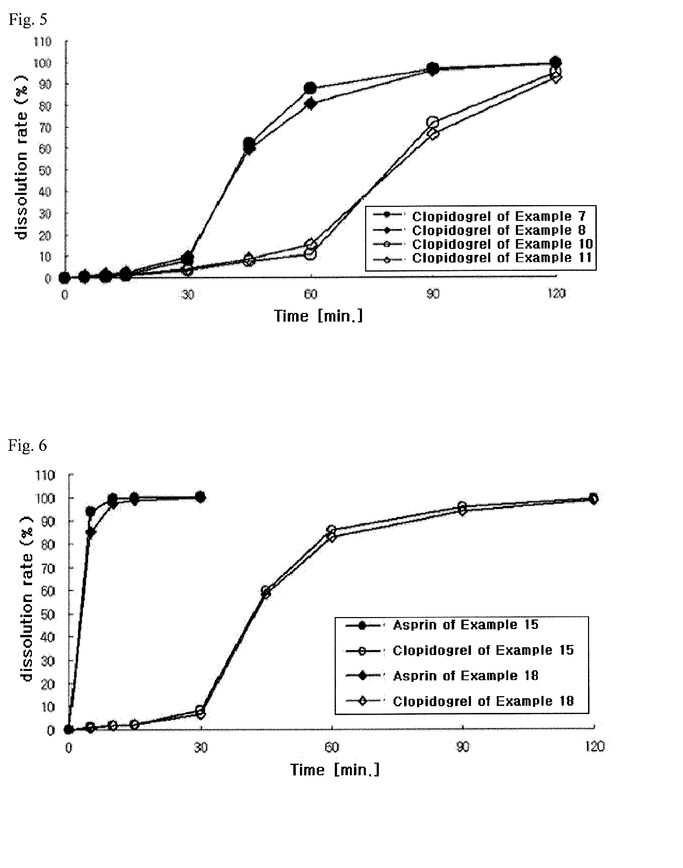

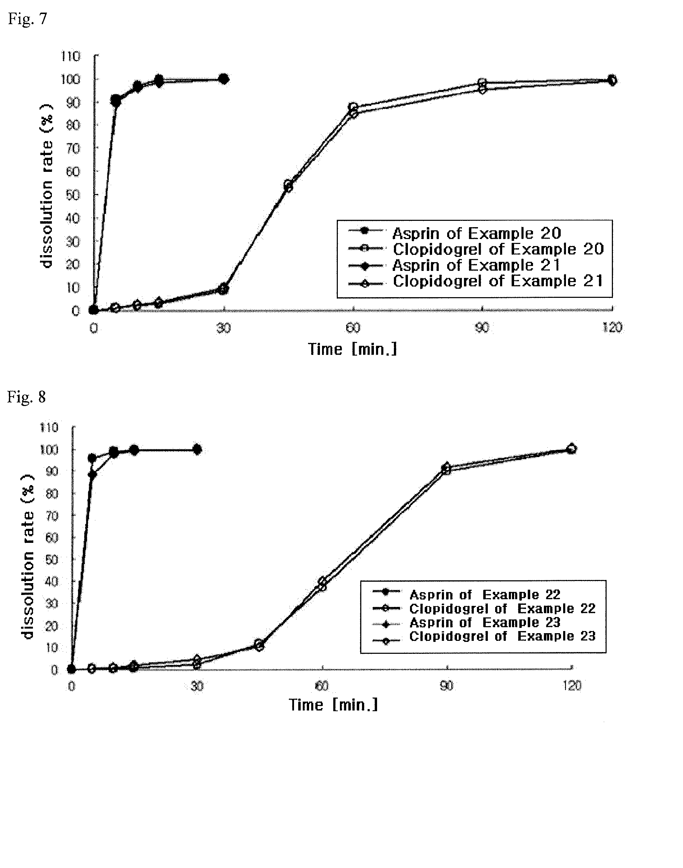

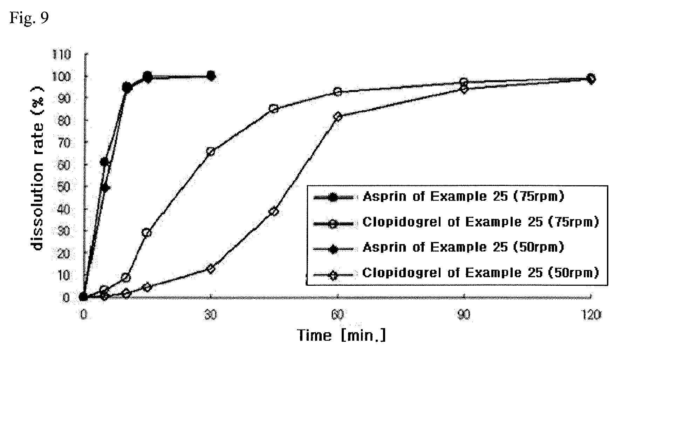

FIG. 1 is a graph showing a dissolution rate of aspirin in preparations of Comparative Example 1, and Preparation Examples 2, 3 and 5; FIG. 2 is a graph showing a dissolution rate of clopidogrel in preparations of Comparative Example 2, and Preparation Examples 8, 11 and 12; FIG. 3 is a graph showing a dissolution rate of aspirin in combination preparations of Examples 1, 6, 10 and 12; FIG. 4 is a graph showing a dissolution rate of clopidogrel in combination preparations of Examples 1, 6, 10 and 12; FIG. 5 is a graph showing a dissolution rate of clopidogrel in combination preparations of Examples 7, 8, 10 and 11; FIG. 6 is a graph showing a dissolution rate of clopidogrel and aspirin in combination preparations of Examples 15 and 18; FIG. 7 is a graph showing a dissolution rate of clopidogrel and aspirin in combination preparations of Examples 20 and 21; and FIG. 8 is a graph showing a dissolution rate of clopidogrel and aspirin in combination preparations of Examples 22 and 23. FIG. 9 is a graph showing a dissolution rate of clopidogrel and aspirin according to revolutions per minute of the paddle (50 rpm, 75 rpm), in a combination preparation of Example 25.

In FIGS. 1 to 9, the x-axis represents the time series, and the y-axis represents the dissolution rate (%).

From FIGS. 1 and 2, it was possible to confirm the dissolution profiles of the clopidogrel-containing delayed-release preparation and the aspirin-containing immediate-release preparation which were applied to the production of the combination preparations of the present invention.

As shown in FIGS. 1 and 2, aspirin exhibited an 85% or higher release at 15 minutes, whereas clopidogrel exhibited a 10% release starting after 15 minutes and an 85% release at 90 minutes, that is, an 85% or higher release was achieved, thus finishing the release thereof.

As can be seen from FIGS. 1 and 2, aspirin of Preparation Examples 2, 3 and 5 exhibited a rapid dissolution rate unlike control drugs. Such rapid dissolution of the aspirin ingredient could minimize the occurrence of gastrointestinal disorders, and it was confirmed that rapid dissolution is possible even when a buffer is contained. In addition, the purpose of chronotherapeutic release of the present invention could be achieved by confirming that the clopidogrel-containing delayed-release compartment exhibits the release of the active ingredient at a time-lag interval of at least 20 minutes.

As shown in FIGS. 3 and 4, in the case of Examples 1 and 6, clopidogrel exhibited a 10% release starting after 15 minutes and an 85% release at 90 minutes, that is, an 85% or higher release was achieved, thus finishing the release thereof. In Examples 10 and 12, clopidogrel exhibited a 10% release starting after 30 minutes and an 85% release at 120 minutes, that is, an 85% or higher release was achieved, thus finishing the release thereof.

From these results, it is believed that the combination preparation of the present invention can exhibit favorable effects by such a manner that disintegration of aspirin after administration thereof is rapidly complete, and therefore aspirin is present in an aqueous solution state in the gastrointestine, whereby gastric absorption of the drug can be maximized while minimizing the gastrointestinal disorders occurring due to contact of solid ingredients with stomach walls. In addition, it is believed that the combination preparation of the present invention can exhibit a complementary anti-platelet aggregation action since aspirin is first released and absorbed and at a low dose, activates a cytochrome P450 2C19 enzyme, and thereafter delayed-released clopidogrel can produce a clopidogrel active metabolite by the action of the activated cytochrome P450 2C19 enzyme. Further, effects of the inventive combination preparation depending on the type of formulations can be confirmed from FIGS. 3 and 4. As can be confirmed from the results, characteristics of the aspirin-containing prior-release compartment exhibited no significant difference in dissolution rate, even with different types of formulations such as single tablet, double-layered tablet, and press-coated tablet. However, the clopidogrel-containing delayed-release compartment exhibited a difference in dissolution rate, depending on the type of formulations. The formulation, like a press-coated tablet, showing no external exposure due to the presence of the clopidogrel ingredient inside the tablet, exhibited a longer time-lag interval, as compared to the formulation which is in exposure to the outside, like a single tablet or double-layered tablet. However, such a difference was intended by the present invention, and it can be designed such that individual active ingredients are released at a time interval.

As confirmed from FIG. 5, when the clopidogrel/aspirin-containing combination preparation in accordance with the present invention was prepared using clopidogrel besylate instead of clopidogrel hydrogen sulphate, there was substantially no difference between both formulations. However, it was confirmed that there is a slight difference in solubility depending on addition salts of clopidogrel. However, time-lag release of clopidogrel intended by the present invention could be achieved irrespective of the addition salt type of clopidogrel.

As shown in FIGS. 6, 7 and 8, the combination preparations of the present invention exhibited no effects of an increased or decreased aspirin or clopidogrel dose (irrespective of dosage forms) on prior release of the aspirin ingredient and delayed release of the clopidogrel ingredient which are sought by the present invention. That is, it was possible to produce preparations containing various ingredients such that they can be administered depending on the condition of patients and desired applications.

According to FIG. 9, the combination preparation of the present invention can provide sequential release of aspirin and clopidogrel by means of a triple-layered tablet formulation, and it can be confirmed that there is a release time interval between two ingredients, even when a rotational frequency of the dissolution profile test paddle is increased.

### Experimental Example 3: Stability test - accelerated & long-term storage tests

The preparations obtained in Examples above were subjected to accelerated and long-term storage tests according to "Stability Testing of New Drug Substances and Products, ICH Guideline Q1A (R2)".

In a specific test method, the pharmaceutical products prepared in Preparation Examples and Examples above were packaged in high-density polyethylene bottles and stored in a constant temperature and humidity chamber under the test conditions of temperature of 40±2°C/relative humidity of 75±5% (accelerated test conditions) and temperature of 25±2°C/relative humidity of 60±5% (long-term storage test conditions). The stored products were taken out at a given interval of time and subjected to a quality test.

The results of the stability test are given in Tables 5 and 6. Table 5 shows the results of the accelerated stability test (40±2°C/relative humidity 75±5%), and Table 6 show the results of the long-term stability test (25±2°C/relative humidity 60±5%).

**[Table 5]**

| Accelerated stability test (40±2°C/relative humidity 75±5%) | | | | | | |
|---|---|---|---|---|---|---|
| | Stability test (accelerated test conditions) | | | | | |
| | Aspirin content (wt%) | | | Clopidogrel content (wt%) | | |
| | Initial | After 4 weeks | Variation | Initial | After 4 weeks | Variation |
| Comparative Example 1 | 99.4 | 98.5 | -0.9 | - | - | - |
| Comparative Example 2 | - | - | - | 99.9 | 98.1 | -1.8 |
| Preparation Example 2 | 99.4 | 98.7 | -0.7 | - | - | - |
| Preparation Example 3 | 99.3 | 98.8 | -0.5 | - | - | - |
| Preparation Example 5 | 99.9 | 98.9 | -1.0 | - | - | - |
| Preparation Example 8 | - | - | - | 99.1 | 99.2 | +0.1 |
| Preparation Example 11 | - | - | - | 99.8 | 98.5 | -1.3 |
| Example 2 | 101.1 | 99.6 | -1.5 | 98.9 | 98.6 | -0.3 |
| Example 10 | 100.6 | 99.4 | -1.2 | 99.6 | 99.2 | -0.4 |
| Example 12 | 100.2 | 99.6 | -0.6 | 100.6 | 99.8 | -0.8 |
| Example 25 | 100.4 | 99.8 | -0.6 | 99.8 | 99.2 | -0.6 |

**[Table 6]**

| Long-term stability test (25±2°C/relative humidity 60±5%) | | | | | | |
|---|---|---|---|---|---|---|
| | Stability test (long-term storage test conditions) | | | | | |
| | Aspirin content (wt%) | | | Clopidogrel content (wt%) | | |
| | Initial | After 6 months | Variation | Initial | After 6 months | Variation |
| Comparative Example 1 | 99.4 | 99.1 | -0.3 | - | - | - |
| Comparative Example 2 | - | - | - | 99.9 | 99.1 | -0.8 |
| Preparation Example 2 | 99.4 | 99.2 | -0.2 | - | - | - |
| Preparation Example 3 | 99.3 | 98.9 | -0.4 | - | - | - |
| Preparation Example 5 | 99.9 | 99.4 | -0.5 | - | - | - |
| Preparation Example 8 | - | - | - | 99.1 | 99.2 | +0.1 |
| Preparation Example 11 | - | - | - | 99.8 | 99.5 | -0.3 |
| Example 2 | 101.1 | 100.7 | -0.4 | 98.9 | 98.7 | -0.2 |
| Example 10 | 100.6 | 100.1 | -0.5 | 99.6 | 99.5 | -0.1 |
| Example 12 | 100.2 | 99.8 | -0.4 | 100.6 | 100.2 | -0.4 |
| Example 25 | 100.4 | 100.1 | -0.3 | 99.8 | 99.6 | -0.2 |

As can be seen from Tables 5 and 6, the combination preparations of clopidogrel and aspirin in accordance with the present invention exhibited excellent results in the accelerated test and long-term storage test. In particular, the combination preparations of the present invention exhibited no decrease in stability, even when compared to formulations where individual ingredients are present alone. Therefore, the clopidogrel-aspirin combination preparations in accordance with the present invention are expected to achieve long-term storability while securing the safety and effectiveness.

### Experimental Example 4: Uniformity of contents test

The tablets obtained in Examples above were tested according to a content uniformity test method among general test methods described in the Korean Pharmacopoeia (8th edition). The results are given in Table 7. As can be seen from Table 7 below, the clopidogrel-aspirin combination preparations of the present invention were not at all affected by content variations according to the type of formulations and the content of main ingredients. Therefore, the clopidogrel-aspirin combination preparations in accordance with the present invention have substantially no variation upon the production thereof in the industrial field, provides superior therapeutic effects to patients due to application of uniform pharmaceutical products, and can prevent side effects due to the content imbalance.

**[Table 7]**

| Long-term stability test (25±2°C/relative humidity 60±5%) | | | | | | |
|---|---|---|---|---|---|---|
| | Stability test (long-term storage test conditions) | | | | | |
| | Aspirin content (wt%) | | | Clopidogrel content (wt%) | | |
| | Initial | After 6 months | Variation | Initial | After 6 months | Variation |
| Comparative Example 1 | 99.4 | 99.1 | -0.3 | - | - | - |
| Comparative Example 2 | - | - | - | 99.9 | 99.1 | -0.8 |
| Preparation Example 2 | 99.4 | 99.2 | -0.2 | - | - | - |
| Preparation Example 3 | 99.3 | 98.9 | -0.4 | - | - | - |
| Preparation Example 5 | 99.9 | 99.4 | -0.5 | - | - | - |
| Preparation Example 8 | - | - | - | 99.1 | 99.2 | +0.1 |
| Preparation Example 11 | - | - | - | 99.8 | 99.5 | -0.3 |
| Example 2 | 101.1 | 100.7 | -0.4 | 98.9 | 98.7 | -0.2 |
| Example 10 | 100.6 | 100.1 | -0.5 | 99.6 | 99.5 | -0.1 |
| Example 12 | 100.2 | 99.8 | -0.4 | 100.6 | 100.2 | -0.4 |
| Example 25 | 100.4 | 100.1 | -0.3 | 99.8 | 99.6 | -0.2 |

### Experimental Example 4: Efficacy test in animals

Animal tests supporting the effects of the present invention were carried out as described in Table 8 below.

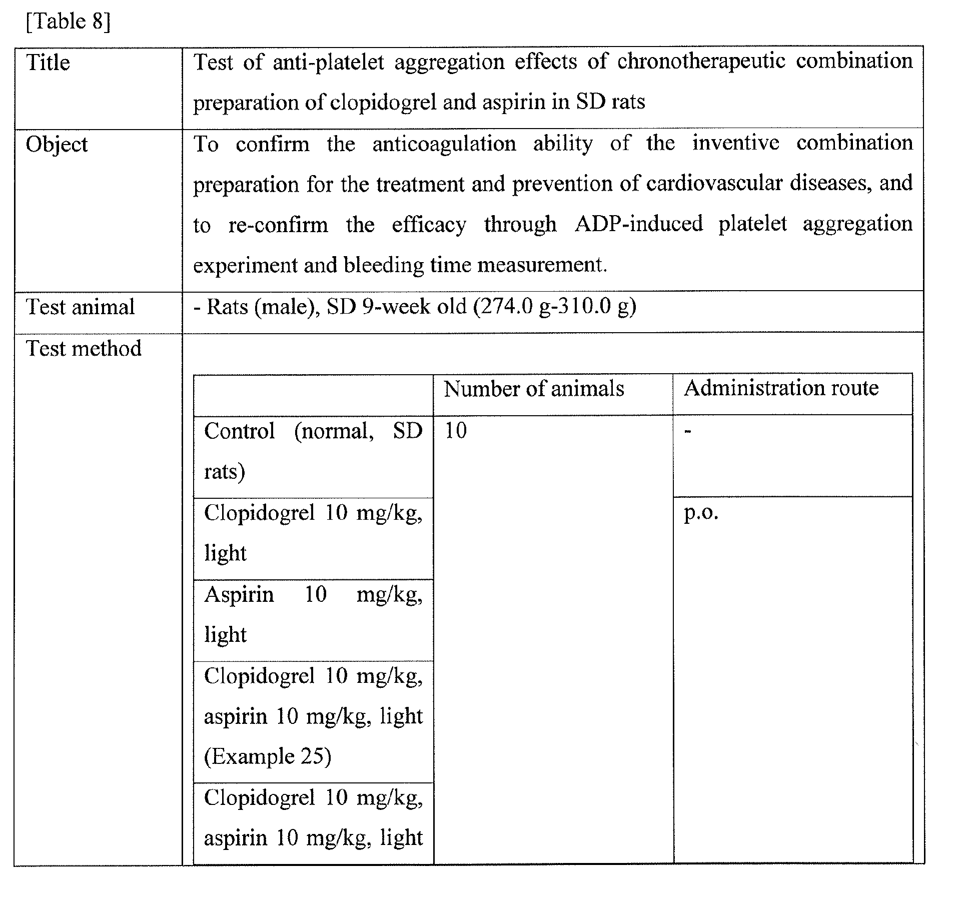

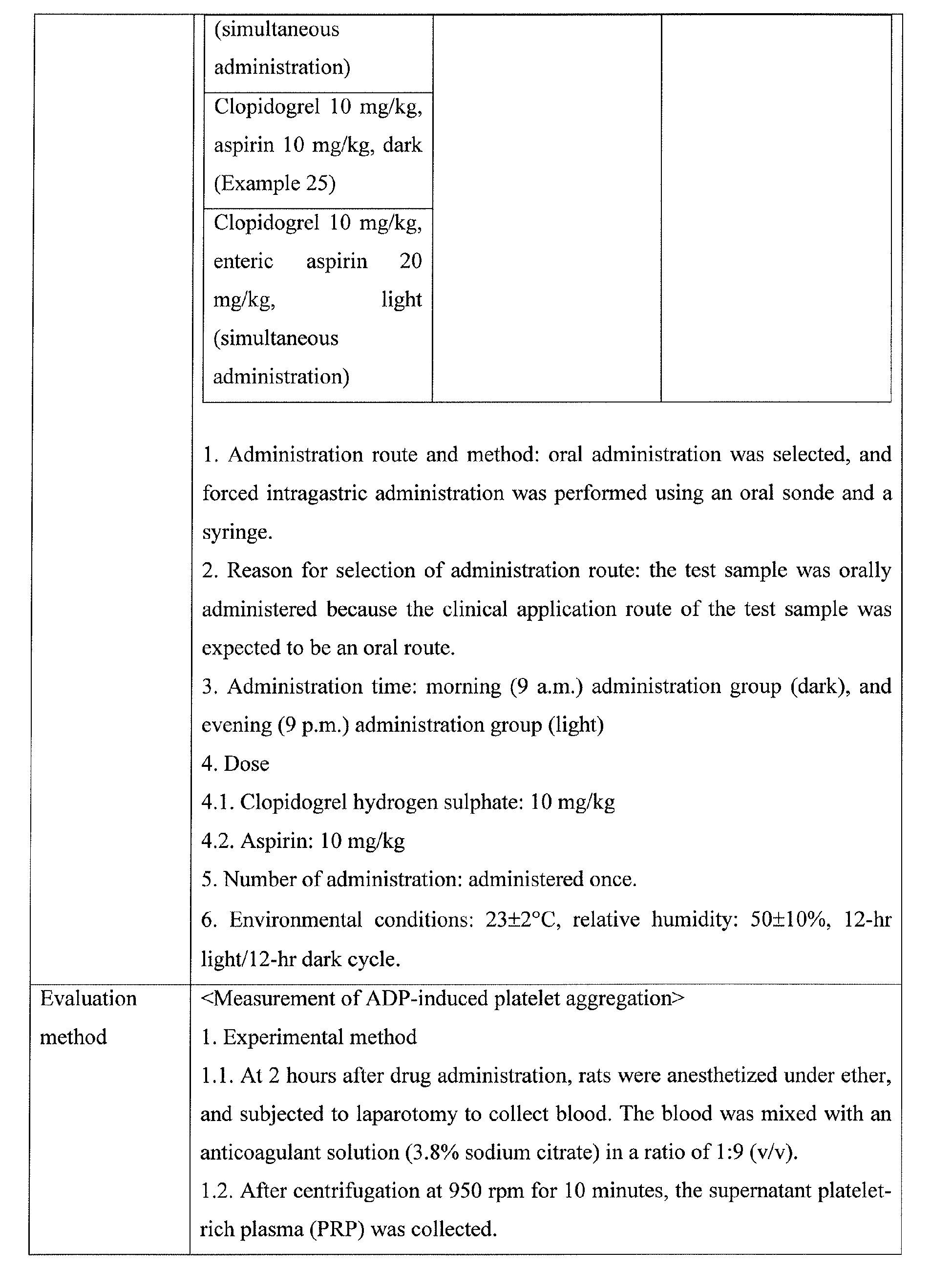

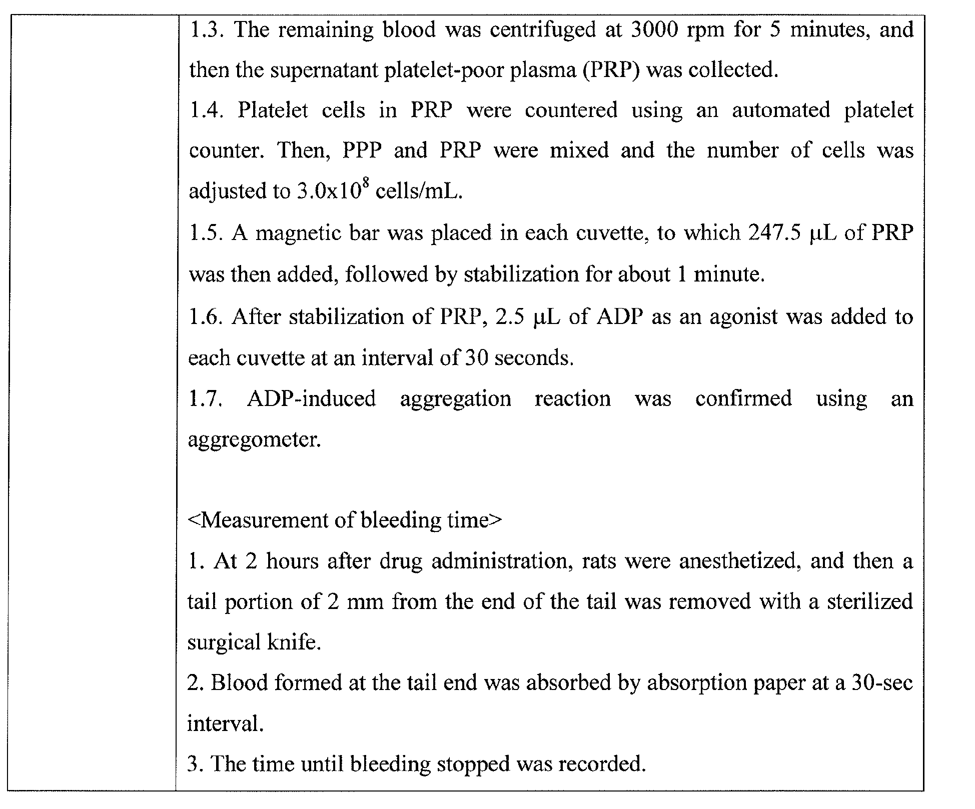

The experimental results are given in Tables 9 and 10 below. Table 9 shows the measurement results of bleeding time in SD rats, and Table 10 shows the results of a measurement experiment of ADP-induced platelet aggregation in SD rats.

As can be seen from Tables 9 and 10, the modified combination preparation in accordance with the present invention exhibited superior effects as compared to single administration of individual ingredients, and chronotherapeutic evening administration exhibited superior effects to simultaneous morning administration. Further, the preparation of the present invention exhibited superior effects despite a lower dose than that of conventionally used enteric aspirin.

**[Table 9]**

| Bleeding time measurement | |
|---|---|
| Test group | Bleeding time |
| Control (normal, SD rats) | 750±468 seconds |
| Clopidogrel 10 mg/kg, light | 1177±729seconds |
| Aspirin 10 mg/kg, light | 930±296 seconds |
| Clopidogrel 10 mg/kg, aspirin 10 mg/kg, light | 2016±1345 seconds |
| (Example 25) | |
| Clopidogrel 10 mg/kg, aspirin 10 mg/kg, light | 1568±983 seconds |
| (simultaneous administration) | |
| Clopidogrel 10 mg/kg, aspirin 10 mg/kg, dark | 1734±852 seconds |
| (Example 25) | |
| Clopidogrel 10 mg/kg, enteric aspirin 20 | 1626±1093 seconds |
| mg/kg, light (simultaneous administration) | |

**[Table 10]**

| Platelet aggregation measurement | |
|---|---|
| Test group | Platelet aggregation |
| Control (normal, SD rats) | 100.0±7.1% |
| Clopidogrel 10 mg/kg, light | 77.1±20.9% |
| Aspirin 10 mg/kg, light | 83.5±16.7% |
| Clopidogrel 10 mg/kg, aspirin 10 mg/kg, light | 59.6±20.3% |
| (Example 25) | |
| Clopidogrel 10 mg/kg, aspirin 10 mg/kg, light | 69.2±7.8% |
| (simultaneous administration) | |
| Clopidogrel 10 mg/kg, aspirin 10 mg/kg, dark | 72.2±10.8% |
| (Example 25) | |
| Clopidogrel 10 mg/kg, enteric aspirin 20 | 71.4±18.6% |
| mg/kg, light (simultaneous administration) | |

### INDUSTRIAL APPLICABILITY

The combination preparation of clopidogrel and aspirin in accordance with the present invention may exhibit superior anti-platelet aggregation effects through the application of the xenobiotic theory and chronotherapeutic theory to a formulation technique of a pharmaceutical preparation, as compared to administration of individual ingredients as single drugs, or concurrent administration of single drugs of individual ingredients or administration of a simple combination preparation of individual ingredients, thereby exhibiting preventive and therapeutic effects against cardiovascular diseases.

Accordingly, the combination preparation of the present invention may be excellent in clinical therapeutic effects and therefore can exhibit the same results even at a dose lower than a conventional normal dose. That is, a dose of main ingredients required for the prevention and treatment of cardiovascular diseases can be reduced and as a result, side effects are significantly reduced and production costs are curtailed.

The combination preparation of the present invention is also advantageous in that it exhibits outstanding anti-platelet aggregation effects despite a reduced dose of aspirin to be administered, in that it converts clopidogrel resistance into clopidogrel sensitivity and thereby prevents serious side effects due to clopidogrel resistance, and in that it can be stored over the longer term since it is stable under common storage conditions.

Finally, the combination preparation of the present invention can exhibit the above-mentioned superior effects by a once-daily administration through various formulations and can therefore improve the medication compliance of patients.

## Claims

1. A combination preparation comprising a prior-release compartment comprising aspirin or a pharmaceutically acceptable salt thereof as a pharmacologically active ingredient, and a delayed-release compartment comprising clopidogrel, an isomer thereof or a pharmaceutically acceptable salt thereof as a pharmacologically active ingredient.

2. The combination preparation according to claim 1, wherein a content of aspirin is in the range of 0.01 to 20 parts by weight, relative to 1 part by weight of clopidogrel.

3. The combination preparation according to claim 1, wherein clopidogrel is released at a time-lag interval of 5 to 120 minutes after aspirin is released.

4. The combination preparation according to claim 1, wherein the pharmaceutically acceptable salt of clopidogrel is clopidogrel hydrogen sulphate or clopidogrel besylate.

5. The combination preparation according to claim 1, wherein the prior-release compartment further includes an immediate-release material.

6. The combination preparation according to claim 5, wherein the immediate-release material is at least one selected from a disintegrant, a foaming agent and a buffer.

7. The combination preparation according to claim 6, wherein the disintegrant is at least one selected from sodium starch glycolate, corn starch, potato starch, pregelatinized starch, bentonite, montmorillonite, veegum, microcrystalline cellulose, hydroxypropylcellulose, carboxymethylcellulose, sodium alginate, alginic acid, croscarmellose sodium, guar gum, xanthan gum, and crosslinked polyvinylpyrrolidone (crospovidone).

8. The combination preparation according to claim 6, wherein the foaming agent is a carbonate-containing inorganic material and organic acid.

9. The combination preparation according to claim 8, wherein the carbonate-containing inorganic material is at least one selected from sodium hydrogen carbonate, sodium carbonate, calcium carbonate, potassium carbonate, magnesium carbonate, calcium hydrogen carbonate and potassium hydrogen carbonate.

10. The combination preparation according to claim 8, wherein the organic acid is at least one selected from citric acid, hydrochloric acid, lactic acid, phosphoric acid, propionic acid, sulfuric acid, tartaric acid, fumaric acid, and malic acid.

11. The combination preparation according to claim 6, wherein the buffer is at least one selected from calcium carbonate, sodium dihydrogen phosphate, sodium monohydrogen phosphate, sodium glutamate, potassium citrate, sodium hydrogen carbonate, sodium citrate, sodium hydroxide, calcium phosphate, calcium hydrogen phosphate, and salts thereof.

12. The combination preparation according to claim 5, wherein the prior-release compartment further comprises at least one additive selected from a diluent, a binder, a glidant, a stabilizer and a film-coating agent.

13. The combination preparation according to claim 12, wherein the diluent is at least one selected from calcium carbonate, calcium phosphate, cellulose, dextrin, dextrose, ethylcellulose, fructose, glyceryl palmitostearate, maltose, sucrose, starch, microcrystalline cellulose, lactose, glucose, mannitol, alginate, alkaline earth metal salt, clay, polyethylene glycol, and dicalcium phosphate.

14. The combination preparation according to claim 12, wherein the binder is at least one selected from alginic acid, carbomer, sodium carboxymethylcellulose, dextrin, ethylcellulose, hydroxyethylcellulose starch, hydroxyethylmethylcellulose, methylcellulose, polyethylene oxide, poloxamer, microcrystalline cellulose, mannitol, lactose, polyethylene glycol, polyvinylpyrrolidone, hydroxypropylmethylcellulose, hydroxypropylcellulose, natural gum, synthetic gum, copovidone, and gelatin.

15. The combination preparation according to claim 12, wherein the glidant is at least one selected from talc, stearic acid and salts thereof, sodium lauryl sulfate, hydrogenated vegetable oil, sodium benzoate, sodium stearyl fumarate, glyceryl monostearate, and polyethylene glycol.

16. The combination preparation according to claim 12, wherein the stabilizer is at least one selected from butylated hydroxytoluene (BHT), butylated hydroxyanisole (BHA), ascorbic acid, tocopherol, and edetic acid (EDTA).

17. The combination preparation according to claim 12, wherein the film-coating agent is at least one selected from gelatin, methylcellulose, hydroxypropylcellulose, hydroxypropylmethylcellulose, polyethylene glycol, shellac, ethylcellulose, methylhydroxyethylcellulose, hydroxyethylcellulose, sodium carboxymethylcellulose, polyvinyl alcohol, polyvinylpyrrolidone, a vinylpyrrolidone/vinyl acetate polymer, an ethyl acrylate/methyl methacrylate/trimethyl ammonium chloride ethyl methacrylate copolymer, a methyl methacrylate/ethyl acrylate copolymer, and polyvinylacetyl dimethylamino acetate.

18. The combination preparation according to claim 1, wherein the prior-release compartment is a fast-disintegrating tablet further comprising a disintegrant in addition to an active ingredient.

19. The combination preparation according to claim 1, wherein the prior-release compartment is an effervescent tablet further comprising a foaming agent in addition to an active ingredient.

20. The combination preparation according to claim 1, wherein the prior-release compartment is a buffer tablet further comprising a buffer in addition to an active ingredient.

21. The combination preparation according to any one of claims 1 to 5, wherein the delayed-release compartment further comprises a release-controlling material in addition to clopidogrel.

22. The combination preparation according to claim 21, wherein the delayed-release compartment contains 0.01 to 10 parts by weight of the release-controlling material, based on 1 part by weight of clopidogrel.

23. The combination preparation according to claim 21, wherein the release-controlling material is at least one selected from a water-soluble polymer, a water-insoluble polymer, and an enteric polymer.

24. The combination preparation according to claim 23, wherein the water-soluble polymer is at least one selected from the group consisting of a water-soluble cellulose ether selected from methylcellulose, hydroxypropylcellulose and hydroxypropylmethylcellulose, a water-soluble polyvinyl derivative selected from polyvinylpyrrolidone and polyvinyl alcohol, and an alkylene oxide polymer selected from polyethylene glycol and polypropylene glycol.

25. The combination preparation according to claim 23, wherein the water-insoluble polymer is at least one selected from polyvinyl acetate, a polymethacrylate copolymer, a poly(ethyl acrylate/methyl methacrylate) copolymer, an ethyl acrylate/methyl methacrylate/trimethylaminoethyl methacrylate copolymer, ethylcellulose, cellulose ester, cellulose ether, cellulose acylate, cellulose diacylate, cellulose triacylate, cellulose acetate, cellulose diacetate, and cellulose triacetate.

26. The combination preparation according to claim 23, wherein the enteric polymer is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/ethyl methacrylate copolymer, a methacrylic acid/ethyl acrylate copolymer, a methyl acrylate/methacrylic acid/octyl acrylate copolymer, vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer, polyvinyl alcohol phthalate, polyvinylacetal phthalate, polyvinylbutyrate phthalate, and polyvinylacetacetal phthalate.

27. The combination preparation according to claim 21, wherein the delayed-release compartment further comprises at least one additive selected from a diluent, a binder, a disintegrant, a glidant, a stabilizer, and a film-coating agent which are pharmaceutically acceptable.

28. The combination preparation according to claim 1, wherein the combination preparation is any one of a pellet, an uncoated tablet, a coated tablet with a film-like coating layer, a multi-layered tablet, a press-coated tablet and a capsule.

29. The combination preparation according to claim 28, wherein the pellet is made up of a clopidogrel coating layer formed of a delayed-release compartment on the sugar sphere surface, and an aspirin coating layer formed of a prior-release compartment enclosing the clopidogrel layer.

30. The combination preparation according to claim 28, wherein the multi-layered tablet is in the form of a double-layered tablet including an aspirin layer formed of a prior-release compartment and a clopidogrel layer formed of a delayed-release compartment.

31. The combination preparation according to claim 28, wherein the multi-layered tablet is in the form of a triple-layered tablet including an aspirin layer formed of a prior-release compartment, a clopidogrel layer formed of a delayed-release compartment, and a placebo layer which does not contain a pharmaceutical ingredient.

32. The combination preparation according to claim 28, wherein the press-coated tablet is made up of a clopidogrel inner core formed of a delayed-release compartment and an aspirin outer layer formed of a prior-release compartment.

33. The combination preparation according to claim 32, wherein the clopidogrel inner core further comprises ethylcellulose as a release-controlling material, in addition to clopidogrel.

34. The combination preparation according to claim 33, wherein the clopidogrel inner core further comprises hydroxypropylmethylcellulose as a release-controlling material.

35. The combination preparation according to claim 28, wherein the capsule is in the form of a capsule containing a particle, granule, pellet, or tablet formed of a delayed-release compartment and a particle, granule, pellet, or tablet formed of a prior-release compartment.

36. The combination preparation according to claim 28, wherein a content of the coating layer is in the range of 0.5 to 20 % by weight, based on the total weight of the coated tablet.

37. The combination preparation according to any one of claims 1 to 5, wherein the preparation is in the form of a kit including a delayed-release compartment and a prior-release compartment.

38. The combination preparation according to any one of claims 1 to 5, wherein a content of clopidogrel per unit preparation is in the range of 35.0 to 500.0 mg, and a content of aspirin is in the range of 10 to 1000 mg.

39. The combination preparation according to any one of claims 1 to 5, wherein the preparation is for evening administration.

40. A method for the prevention and treatment of a cardiovascular disease, comprising administering to a subject a combination preparation of any one of claims 1 to 5 once a day, between 5 p.m. and 11 p.m.

41. A method for the prevention and treatment of a cardiovascular disease, comprising administering aspirin to a subject, and then administering clopidogrel 5 to 120 minutes later.
